# EUROPÄISCHE PATENTANMELDUNG

(11) **EP 4 635 456 A2**
(43) Veröffentlichungstag der Anmeldung: **22.10.2025**
(21) Anmeldenummer: 25181763.1
(22) Anmeldetag: 06.05.2022
(51) Int. Cl.: A61F 2/915

(54) **SELBSTEXPANDIERENDER STENT MIT GESTUFTEM RADIALKRAFTPROFIL**

(30) Priorität: 04.06.2021 DE 102021114450; 04.06.2021 DE 102021114444
(62) Teilanmeldung aus: 22728374.4
(71) Anmelder: Angiolutions GmbH, 30179 Hannover (DE)
(72) Erfinder: Raaz, Uwe, 04105 Leipzig (DE); Nißl, Thomas, 37318 Mackenrode (DE); Schellinger, Isabel Nahal, 04105 Leipzig (DE)
(74) Vertreter: Eisenführ Speiser

(57) **Zusammenfassung**

Die Erfindung betrifft einen selbstexpandierenden flexiblen intravaskulären Stent (1) zum Einsatz in ein Gefäß, mit zwei oder mehr axial miteinander verbundenen Ringsegmenten (2, 2a), die jeweils aus einer Mehrzahl an mäanderförmig über Bögen (7) miteinander verbundener Stegen (5, 5a) gebildet sind. Der Stent (1) hat einen gecrimpten Zustand mit reduziertem Durchmesser und einen expandierten Zustand mit einem Nominaldurchmesser (DN). Die Ringsegmente (2, 2a) weisen in einem ersten Durchmesserbereich (D1) kleiner als der Nominaldurchmesser (DN) eine erste Expansionscharakteristik, und in einem zweiten Durchmesserbereich (D2) größer als der Nominaldurchmesser (DN) eine zweite Expansionscharakteristik auf, die verscheiden ist von der ersten Expansionscharakteristik. Die Erfindung betrifft ferner die Verwendung eines Stents sowie ein Herstellungsverfahren für einen Stent.

## Beschreibung

Die Erfindung betrifft einen selbstexpandierenden Stent mit einer Mehrzahl von Ringsegmenten mit mäandrierendem Verlauf, die von einer Mehrzahl von Stegen gebildet werden, wobei die Ringsegmente mit benachbarten Ringsegmenten verbunden sind. Der Stent ist für endovaskuläre Anwendungen bestimmt.

Stents zur Erweiterung verengter Blutgefäße und zur Verankerung von Gefäßimplantaten in einem Blutgefäß sind bekannt und vielfach beschrieben. Man unterscheidet zwischen selbstexpandierenden Stents aus einer Formgedächtnislegierung und ballonexpandierbaren Stents, in der Regel aus einem medizinischen Stahl, die beide über endovaskuläre Katheter implantiert werden.

Zumeist bestehen Stents aus einer Vielzahl von Ringsegmenten, die über Verbindungsstege mit benachbarten Ringsegmenten verbunden sind. Die Ringsegmente haben häufig einen mäandrierenden oder zickzack-förmigen Verlauf und werden von Stegen gebildet, die bei der Expansion auseinander gespreizt werden. Die einzelnen Stege sind durch Bögen miteinander verbunden. Der bei dieser Technik auftretenden Längenverkürzung kann durch ein geeignetes Design und/oder eine geeignete Anordnung der Verbindungsstege entgegengewirkt werden.

Das Eröffnen und radiale Aufdehnen eines Blutgefäßes kann erhebliche Radialkräfte erfordern, die von dem jeweiligen Stent aufgebracht werden müssen. Bei selbstexpandierenden Stents erfordert dies eine entsprechende Stärke der Stege. Bei ballonexpandierbaren Stents werden die Radialkräfte über den zur Expansion verwandten Ballon aufgebracht. Die bei selbstexpandierenden Stents nach der Stentexpansion auf die Wand des Gefäßes permanent wirkende chronische Auswärtskraft (chronic outward force, COF) sorgt für eine stabile Verankerung des Stents an der Gefäßwand und wirkt zudem der Tendenz des Gefäßes, sich wieder zusammenzuziehen (recoil) entgegen.

Die COF eines Stents stellt eine permanente mechanische Belastung der Gefäßwand dar, die in kompensatorische Gefäßwachstumsprozesse mit einer progredienten Zunahme des Gefäßdurchmessers münden kann - als adaptives Gefäßremodeling bekannt. In der Folge lockert sich der nach der Implantation hergestellte feste Verbund von Gefäß und Stent.

Dieses Ziel wird allgemein mit einem erfindungsgemäßen selbstexpandierenden Stent erreicht, der über ein (vom Expansionsgrad des Stents abhängiges) gestuftes Radialkraftprofil verfügt. Dabei expandiert der Stent bis zum Erreichen eines spezifischen Nominaldurchmessers zunächst mit hoher prädefinierbaren ersten Radialkraft (F1) bzw. auf das Niveau der prädefinierbaren ersten Radialkraft (F1). Jenseits des Nominaldurchmessers erlaubt der Stent eine weitere Expansion bis zum Erreichen eines spezifischen Maximaldurchmessers. Die Differenz zwischen Nominal- und Maximaldurchmesser entspricht hierbei der "Expansionsreserve" des Stents. Tritt der Stent von seinem Nominaldurchmesser in die Expansionsreserve, nimmt seine Radialkraft sprunghaft (stufenartig) auf ein deutlich geringeres Radialkraftniveau einer prädefinierbaren zweiten Radialkraft (F2) ab, das zum Erreichen des Maximaldiameters wirkt.

Über die Implantation des Stents wird zunächst hohe Radialkräfte/COF (F1) erzeugt, um eine Dehnung des Gefäßes auf einen gewünschten Zieldurchmesser zu erreichen, und dabei ein enger Kontakt des Stents zur Gefäßwand hergestellt, um dadurch z.B. eine Migration des Stents oder - im Falle von gecoverten Stents - einen Blutfluss zwischen Stent und Gefäßwand (Leckage) zu verhindern. Dem Zieldurchmesser des Gefäßes entspricht der Nominaldurchmesser des Stents nach der Aufweitung.

Nach dem Einwachsen des Stents in die Gefäßwand wird jedoch ein Lockern des Stents durch weiteres progressives Wachstum des Durchmessers des Zielgefäßes (adaptives Remodeling) verhindert. Daher kann der Stent einem weiteren Gefäßwachstum jenseits seines Nominaldurchmessers zwar folgen, jedoch übt er nur noch eine sehr geringe Radialkraft/COF (F2) aus. Der Stent folgt also nach der Implantation einer Gefäßerweiterung nur noch mit geringer eigener COF (aktiv) oder rein passiv und stimuliert kein weiteres Gefäßwachstum mehr. Die Erfindung schlägt einen selbstexpandierenden flexiblen intravaskulären Stent mit wenigstens einem, vorzugsweise zwei oder mehr axial miteinander verbundenen Ringsegmenten, vor, die jeweils aus einer Mehrzahl an mäanderförmig über Bögen miteinander verbundenen Stegen gebildet sind. Der Stent hat einen gecrimpten Zustand mit reduziertem Durchmesser und einen expandierten Zustand mit einem Nominaldurchmesser.

Die Ringsegmente weisen in einem ersten Aspekt vorzugsweise in einem ersten Durchmesserbereich kleiner als der Nominaldurchmesser eine erste Expansionscharakteristik und in einem zweiten Durchmesserbereich größer als der Nominaldurchmesser eine zweite Expansionscharakteristik auf, die verschieden ist von der ersten Expansionscharakteristik ist.

Während herkömmliche selbstexpandierende Stents lediglich bis zum Nominaldurchmesser expandieren können und für den Fall eines Gefäßremodelings oberhalb des Nominaldurchmessers nicht mehr weiter mitexpandieren können, wird erfindungsgemäß ein Stent vorgeschlagen, der auch oberhalb des Nominaldurchmessers expandieren kann. Oberhalb des Nominaldurchmessers nämlich im zweiten Durchmesserbereich expandiert der Stent vorzugsweise mit der zweiten Expansionscharakteristik.

Hierbei ist vorzugsweise vorgesehen, dass die erste Expansions-Charakteristik eine Expansion mit einer ersten dauerhaften Auswärtskraft (COF1) und die zweite Expansions-Charakteristik eine Expansion mit einer zweiten dauerhaften Auswärtskraft (COF2) bewirkt, die geringer ist als die erste dauerhafte Auswärtskraft (COF1).

Auf diese Weise kann ein gestuftes Radialkraftprofil erreicht werden, welches ein Expandieren des Stents bis zum Nominaldurchmesser und Aufdehnen des entsprechenden Gefäßes zum Nominaldurchmesser ermöglicht, jenseits des Nominaldurchmessers aber nur noch ein Mitwachsen des Stents erlaubt, der in diesem Bereich nur noch eine sehr geringe Radialkraft bzw. dauerhafte Auswärtskraft aufbringt. Die Radialkraft bzw. dauerhafte Auswärtskraft (COF2) im zweiten Durchmesserbereich, das heißt im Bereich jenseits des Nominaldurchmessers bis zum Maximaldurchmesser ist vorzugsweise so gewählt, dass sie möglichst klein ist und der Gefäßwand beim adaptiven Remodeling folgt. Die Radialkraft bzw. die zweite dauerhafte Auswärtskraft (COF2) im zweiten Durchmesserbereich sollte vorzugsweise so gewählt sein, dass ein aktives weiteres Aufdehnen des Gefäßes nicht stattfindet, sondern die Radialkraft lediglich derart gewählt ist, dass der Stent weiterhin dicht an der inneren Oberfläche des Gefäßes anliegt und diesem folgt. Der Stent soll also im Bereich größer des Nominaldurchmessers lediglich eine derartige Kraft aufbringen, die einen Kontakt zwischen Stent und Gefäßwand sicherstellt. Die Kraft sollte so gewählt sein, dass nicht das Gefäß beim Remodeling an dem Stent "ziehen" muss, sondern idealerweise durch den Stent möglichst wenig physisch auf das Gefäß eingewirkt wird. Mit anderen Worten, anstatt oder zusätzlich zu der ersten und zweiten Expansions-Charakteristik kann der erfindungsgemäße Stent auch über die ersten und zweiten Radialkräfte F1, F2 bzw. COF bzw. Radialkraftniveaus, auf die die Radialkraft im ersten und zweiten Durchmesserbereich abfällt, beschrieben werden. Weiterhin kann der erfindungsgemäße Stent anstatt oder zusätzlich zu der ersten und zweiten Expansions-Charakteristik über den Radialkraftverlauf bei Expansion des Stents beschrieben werden, der wenigstens einen Knick, wenigstens einen, vorzugsweise zwei, Wendepunkte oder wenigstens eine Stufe aufweist. Erfindungsgemäß lässt die vom Stent bewirkte Radialkraft jenseits des Nominaldurchmessers stark nach und fällt auf ein sehr geringes Niveau ab, vorzugsweise derart, dass ein Gefäßremodeling nicht oder nur in sehr geringem Maße stattfindet.

Vorzugsweise beträgt der zweite Durchmesserbereich wenigstens 10% eines Maximaldurchmessers Dmax. Der zweite Durchmesserbereich macht also 10% oder mehr der Gesamtexpansion aus. Vorzugsweise beträgt der zweite Durchmesserbereich wenigstens 15%, 20%, 25%, 30%, 40%, 50%, 60%, oder 70% des Maximaldurchmessers. Dabei ist vorzugsweise vorgesehen, dass der zweite Durchmesserbereich höchstens 90% des Maximaldurchmessers Dmax umfasst, vorzugsweise 80%, weiter vorzugsweise 75%, 70%, 65%, 60%, 55%, oder 50%.

Die Ringsegmente weisen vorzugsweise in einem ersten Durchmesserbereich kleiner als der Nominaldurchmesser eine erste Radial-Steifigkeit und in einem zweiten Durchmesserbereich größer als der Nominaldurchmesser eine zweite Radial-Steifigkeit auf. Die zweite Radial-Steifigkeit ist geringer als die erste Radial-Steifigkeit.

Aufgrund der verschiedenen ersten und zweiten Radial-Steifigkeiten wird im ersten Durchmesserbereich eine zunächst hohe Radialkraft, und im zweiten Durchmesserbereich dann eine geringe Radialkraft erzeugt. Als Steifigkeit wird allgemein ein Verhältnis von Kraft/Verformung verstanden. Im ersten Durchmesserbereich hat der Stent eine höhere Radial-Steifigkeit als im zweiten Durchmesserbereich, also ein höheres Verhältnis Kraft/Verformung, hier konkret Radialkraft/Radialverformung. Dadurch wird beim Expandieren des Stents im ersten Durchmesserbereich eine höhere Kraft pro Verformung als im zweiten Durchmesserbereich bereitgestellt. Im zweiten Durchmesserbereich kann der Stent mit dem Gefäß beim Gefäßremodeling mitwachsen, ohne wesentliche Kräfte auf die Gefäßwand aufzubringen. Vorzugsweise ist die Radial-Steifigkeit wenigstens um den Faktor 2, weiter bevorzugt 2,5, weiter bevorzugt 3, weiter bevorzugt 4, weiter bevorzugt 5, weiter bevorzugt 6, weiter bevorzugt 7, weiter bevorzugt 10, weiter bevorzugt 20 größer als im zweiten Durchmesserbereich.

Vorzugsweise weist der Stent einen ersten Radialkraftbereich in dem ersten Durchmesserbereich bis zum Nominaldurchmesser und einen zweiten Radialkraftbereich in dem zweiten Durchmesserbereich auf. Vorzugsweise ist die erste Radialkraft des Stents in dem ersten Durchmesserbereich in dem ersten Radialkraftbereich und die zweite Radialkraft ist in dem zweiten Durchmesserbereich in dem zweiten Radialkraftbereich. Vorzugsweise weist der Stent einen ersten Radialkraft-Abfall in dem ersten Durchmesserbereich bis zum Nominaldurchmesser und einen zweiten Radialkraft-Abfall in dem zweiten Durchmesserbereich auf. Im ersten Durchmesserbereich fällt die Radialkraft von einem Maximalwert vom gecrimpten Zustand auf ein erstes Plateau bis zum Nominaldurchmesser ab. Wird der Stent über den Nominaldurchmesser hinaus expandiert bzw. expandiert er selbst über den Nominaldurchmesser hinaus, fällt die Radialkraft in einem zweiten Radialkraft-Abfall auf ein zweites Plateau ab. Beim Expandieren nähert sich die Radialkraft im ersten Durchmesserbereich asymptotisch dem ersten Geraden an und im zweiten Durchmesserbereich einer zweiten Geraden an.

Weiter ist bevorzugt, dass die Radialkraft im ersten Durchmesserbereich beim Expandieren auf ein erstes Radialkraftniveau abfällt und im zweiten Durchmesserbereich auf ein zweites Radialkraftniveau abfällt, das geringer ist als das erste Radialkraftniveau. Vorzugsweise ist das erste Radialkraftniveau um einen Faktor größer als das zweite Radialkraftniveau, wobei der Faktor in einem Bereich von 2 bis 20 ist, bevorzugt in einem Bereich von 2 bis 10, weiter bevorzugt von 3 bis 10, weiter bevorzugt von 4 bis 10, weiter bevorzugt von 5 bis 9.

In einer bevorzugten Weiterbildung weist ein Radialkraft-Diameter-Profil des Stents einen Knick oder Sprung auf. Ein Radialkraft-Diameter-Profil stellt den Verlauf der Radialkraft ausgehend von einem gecrimpten Zustand bis zum Maximaldurchmesser über den Durchmesser aufgetragen dar. Bei herkömmlichen Stents hat ein derartiges Radialkraft-Diameter-Profil keinen Knick oder Sprung, stattdessen nimmt die Radialkraft vom gecrimpten Zustand bis zum Maximaldurchmesser im Wesentlichen kontinuierlich, insbesondere degressiv, ab und endet dann abrupt. Der erfindungsgemäße Stent hat ein Radialkraft-Diameter-Profil, welches wenigstens einen Knick aufweist, vorzugsweise wenigsten zwei Knicke. Das Radialkraft-Diameter-Profil weist als Graph aufgetragen wenigstens einen, vorzugsweise zwei Wendepunkte auf. Weiter vorzugsweise hat das Radialkraft-Diameter-Profil im ersten Durchmesserbereich ausgehend vom gecrimpten Zustand beim Expandieren zunächst einen Abschnitt mit einer ersten Steigung, dann einen Abschnitt mit einer zweiten Steigung, und im zweiten Durchmesserbereich ausgehend vom Nominaldurchmesser einen dritten Abschnitt mit einer dritten Steigung und einen vierten Abschnitt mit einer vierten Steigung, wobei die erste Steigung und größer als die zweite Steigung und die vierte Steigung ist, und die dritte Steigung größer als die zweite Steigung und die vierte Steigung ist. Die dritte Steigung kann größer sein als die erste Steigung. Die zweite Steigung kann größer sein als die vierte Steigung. Die erste Steigung ist vorzugsweise wenigstens um einen ersten Steigungsfaktor größer als die zweite Steigung, wobei der erste Steigungsfaktor wenigstens 2,0; 2,5; 3,0; 3,5; 4,0; 4,5; 5,0; 5,5; 6,0; 7,0; 8,0; 9,0; 10,0; 12,0; 15,0 beträgt. Die dritte Steigung ist vorzugsweise wenigstens um einen zweiten Steigungsfaktor größer als die zweite und/oder vierte Steigung, wobei der erste Steigungsfaktor wenigstens 2,0; 2,5; 3,0; 3,5; 4,0; 4,5; 5,0; 5,5; 6,0; 7,0; 8,0; 9,0; 10,0; 12,0; 15,0 beträgt.

Bevorzugt hat das Radialkraft-Diameter-Profil des Stents ausgehend vom gecrimpten Zustand bis zum expandierten Zustand den folgenden Verlauf: im ersten Durchmesserbereich zunächst eine Steigungsabnahme gefolgt von einer Steigungszunahme, dann beim Übertritt in den zweiten Durchmesserbereich eine erneute Steigungsabnahme und vorzugsweise abschließend erneute Steigungszuname. Die erste Steigungsabnahme im ersten Durchmesserbereich verläuft bis vorzugsweise zum Nominaldurchmesser. Bei einer Expansion über den Nominaldurchmesser hinaus folgt dann die erste Steigungszunahme, und dann im zweiten Durchmesserbereich eine zweite Steigungsabnahme. Der Verlauf kann auch als degressiv-progressiv-degressiv beschrieben werden.

Weiter bevorzugt definiert der erste Durchmesserbereich einen ersten Radial-Steifigkeitsprofilabschnitt und der erste Radial-Steifigkeitsprofilabschnitt ist degressiv, regressiv oder linear. Eine degressive oder regressive Steifigkeit bei größer werdendem Durchmesser des Stents sorgt für einen rascheren Kraftabfall beim Expandieren. Vorzugsweise ist die Radialkraft im ersten Durchmesserbereich über wenigstens einen Abschnitt im Wesentlichen konstant oder nur leicht abfallend, und im zweiten Durchmesserbereich ebenfalls im Wesentlichen konstant. Dies kann dadurch erreicht werden, dass die Radialsteifigkeit degressiv oder regressiv ausgebildet ist.

Vorzugsweise ist auch für den zweiten Durchmesserbereich vorgesehen, dass der zweite Durchmesserbereich einen zweiten Radial-Steifigkeitsprofilabschnitt definiert und der zweite Radial-Steifigkeitsprofilabschnitt degressiv, regressiv oder linear ist.

In einer bevorzugten Weiterbildung ist vorgesehen, dass der zweite Radial-Steifigkeitsprofilabschnitt stärker degressiv ist oder eine größere Steigung hat als der erste Radial-Steifigkeitsprofilabschnitt. In einer weiteren bevorzugten Ausführungsform ist vorgesehen, dass der Stent in dem ersten Durchmesserbereich eine erste dauerhafte Radialkraft COF1 (chronic outward force) in einem ersten Radialkraftbereich bereitstellt und in dem zweiten Durchmesserbereich eine zweite dauerhafte Radialkraft COF2 (chronic outward force) in einem zweiten Radialkraftbereich bereitstellt. Die erste dauerhafte Radialkraft ist vorzugsweise um einen Faktor von wenigstens 2, vorzugsweise 4, größer als die zweite dauerhafte Radialkraft oder die zweite dauerhafte Radialkraft beträgt annähernd bzw. nahezu 0. Vorzugsweise beträgt die zweite dauerhafte Radialkraft 10 N oder weniger. Die Radialkraft ist auch abhängig von der axialen Länge des Stents. Die zweite dauerhafte Radialkraft ausgedrückt als längenabhängiger Wert liegt vorzugsweise in einem Bereich von kleiner 1 N / mm, vorzugsweise kleiner 0,5 N / mm, weiter bevorzugt kleiner 0,25 N / mm. Als untere Grenze kommt jeweils ein Wert von 0,001 N / mm in Frage.

In einer bevorzugten Weiterbildung weist jedes Ringsegment über die Ringzirkumferenz verteilt eine Mehrzahl an ersten Zirkumferenzsegmenten und eine Mehrzahl an zweiten Zirkumferenzsegmenten auf, die sich strukturell unterscheiden. Auf diese Weise lassen sich durch die unterschiedlichen ersten und zweiten Zirkumferenzsegmente die verschiedenen Radialkräfte für den ersten und zweiten Durchmesserbereich bereitstellen. Vorzugsweise wirken im ersten Durchmesserbereich die ersten und zweiten Zirkumferenzsegmente, während bei Erreichen des Nominaldurchmessers die ersten Zirkumferenzsegmente vollständig expandiert sind, und lediglich die zweiten Zirkumferenzsegmente weiter wirken, jedoch nur mit einer geringeren Kraft, nämlich der zweiten dauerhaften Auswärtskraft.

Vorzugsweise sind die ersten Zirkumferenzsegmente so ausgebildet, dass sie die erste Radial-Steifigkeit aufweisen oder definieren und die zweiten Zirkumferenzsegmente die zweite Radial-Steifigkeit aufweisen oder definieren. Die zweite Radial-Steifigkeit der zweiten Zirkumferenzsegmente ist vorzugsweise vernachlässigbar gegenüber der ersten Radial-Steifigkeit der ersten Zirkumferenzsegmente. Vorzugsweise definieren die ersten und die zweiten Zirkumferenzsegmente gemeinsam die erste Expansions-Charakteristik im ersten Durchmesserbereich, während im zweiten Durchmesserbereich allein die zweiten Zirkumferenzsegmente expandieren und so die zweite Expansions-Charakteristik durch die zweiten Zirkumferenzsegmente definiert ist.

Bevorzugt ist hierbei, dass die ersten Zirkumferenzsegmente und zweiten Zirkumferenzsegmente benachbarter Ringsegmente jeweils axial zueinander ausgerichtet sind. Das heißt, erste Zirkumferenzsegmente zweier benachbarter Ringsegmente sind axial fluchtend und somit nebeneinander ausgerichtet. Ebenso sind zweite Zirkumferenzsegmente von zwei benachbarten Ringsegmenten axial benachbart und fluchtend zueinander ausgerichtet. Hierdurch kann ein gleichmäßiges Öffnen und Expandieren des Stents erreicht werden, ohne dass Drehkräfte um eine Zentralachse des Stents ausgeübt werden.

Gemäß einer bevorzugten Weiterbildung ist vorgesehen, dass die Stege der ersten Zirkumferenzsegmente eine größere Stärke aufweisen als die Stege der zweiten Zirkumferenzsegmente. Die ersten Zirkumferenzsegmente können auch als "harte Stentsegmente" bezeichnet werden und die zweiten Zirkumferenzsegmente als "weiche Stentsegmente". Unter "harten Stentsegmenten", "harten Zirkumferenzsegmenten" oder "harten Ringsegmenten" können solche verstanden werden, die die erste Radialkraft definieren. Unter "weichen Stentsegmenten", "weichen Zirkumferenzsegmenten" oder "weichen Ringsegmenten" können solche verstanden werden, die die zweite Radialkraft definieren. "Harte Stentsegmente" sind demnach solche, die die erste Radial-Steifigkeit im Wesentlichen definieren und "weiche Stentsegmente" sind solche, die die zweite Radial-Steifigkeit im Wesentlichen definieren.

Ein gestuftes Radialkraftprofil wird insbesondere mit einem Stent der eingangs beschriebenen Art erreicht, bei dem die Ringsegmente von über die Ringzirkumferenz verschiedenartig angeordneten Elementen mit unterschiedlicher Expansionskraft (harte und weiche Elemente) gebildet werden, von denen die harten Elemente bei der Expansion den Nominaldurchmesser definieren und die weichen Elemente eine Expansionsreserve darstellen.

Ein Aufbau der Ringsegmente dieser Art aus harten und weichen Elementen wird beispielhaft mit einem Stent der eingangs beschriebenen Art erreicht, bei dem die Ringsegmente von harten und weichen Stentsegmenten gebildet werden, von denen die harten Stentsegmente bei der Expansion den Nominaldurchmesser definieren und die weichen Stentsegmente eine Expansionsreserve darstellen, wobei die Stege der harten Stentsegmente eine größere Stärke aufweisen als die Stege der weichen Stentsegmente.

Unter einem Ringsegment wird eine quer zur Längsrichtung des Stents verlaufende Abfolge von in expandiertem Zustand zickzack-förmig oder mäandrierend angeordneten Stegen verstanden, die durch Bögen oder Eckpunkte miteinander verbunden sind, wobei vorzugsweise eine Mehrzahl von Ringsegmenten nebeneinander angeordnet ist und den Stent ausbilden. Die Ringsegmente sind vorzugsweise jeweils mit benachbarten Ringsegmenten verbunden, vorzugsweise über Verbindungsstege oder über gemeinsame Eckpunkte, bei randständigen Ringsegmenten aber auch durch direkten Kontakt einander gegenüberstehender Bögen bzw. Eckpunkt. Verbindungsstege können gerade oder gekrümmt verlaufen und an den Bögen der Ringsegmente außen und/oder innen ansetzen. Die Begriffe zick-zack-förmig und mäandrierend werden als synonym verstanden.

Unter Stentsegmenten oder Zirkumferenzsegmenten kann in einer Variante eine Abfolge von Stegen gleicher Stärke verstanden werden, die expandiert zickzack-förmig oder mäandrierend verlaufen. Ein Ringsegment enthält dabei wenigstens ein hartes Stentsegment und wenigstens ein weiches Stentsegment oder ein erstes Zirkumferenzsegment und ein zweites Zirkumferenzsegment. Harte Stentsegmente bzw. erste Zirkumferenzsegmente bestehen vorzugsweise aus einer Mehrzahl von durch Bögen miteinander verbundenen Stegen, die nach der Expansion im Nominaldurchmesser aufgespreizt sind. Weiche Stentsegmente bzw. zweite Zirkumferenzsegmente bestehen in einer Ausführungsform vorzugsweise aus zwei durch einen Bogen miteinander verbundenen Stegen, die im Nominaldurchmesser parallel verlaufen und bei einer Nachexpansion des Gefäßes (remodeling) im Durchmesserbereich größer als der Nominaldurchmesser aufspreizen (Expansionsreserve des Stents). Die einzelnen Stege der Ringsegmente verlaufen vorzugsweise gerade und sind mit benachbarten Stegen immer durch Bögen verbunden. Im nicht expandierten (gecrimpten) Zustand des Stents verlaufen alle Stege in der Regel parallel.

Die Härte bzw. Steifigkeit der Stentsegmente kann durch die Stärke der jeweiligen Stege bestimmt werden. Beispielsweise gibt die Stegbreite die Steifigkeit vor: Je breiter die Stege, desto härter bzw. steifer das Stentsegment. Weiche Stentsegmente können in diesem Fall entsprechend schmaler sein als harte Stentsegmente. Es versteht sich, dass die Härte bzw. Steifigkeit der Stentsegmente auch über ihre Dicke definiert sein kann, wobei sich aber bei der üblichen Laserschneidetechnik, bei der der Stent aus einem Rohr geschnitten wird, die Stegbreite zur Bestimmung der Härte bzw. Steifigkeit anbietet. Durch die unterschiedliche Stegbreite entsteht in einem Ausführungsbeispiel ein abgestuftes Radialkraftprofil/ Expansionsprofil.

Vorzugsweise weisen die erfindungsgemäßen Stents wenigstens zwei zweite Zirkumferenzsegmente (weiche Stentsegmente) pro Ringsegment auf. Dabei können die weichen Stentsegmente in Längsrichtung des Stents linear aufeinanderfolgend angeordnet sein oder aber von Ringsegment zu Ringsegment spiralförmig gegeneinander versetzt. Die zweiten Zirkumferenzsegmente zweier benachbarter Ringsegmente sind vorzugsweise axial benachbart und miteinander fluchtend angeordnet. Vorzugsweise ist in allen Ringsegmenten die gleiche Anzahl zweiter Zirkumferenzsegmente (weicher Stentsegmente) vorhanden. Beispielsweise weist ein Ringsegment zwei bis vier zweite Zirkumferenzsegmente (weiche Stentsegmente auf), bei 12 bis 18 Zirkumferenzsegmenten je Ringsegment insgesamt.

Um ein weiter abgestuftes passives Expansionsverhalten herbeizuführen, können die Ringsegmente weiche Stentsegmente mit Stegen unterschiedlicher Stärke aufweisen. Mit anderen Worten, die zweiten Zirkumferenzsegmente können in einer Weiterbildung derart gestaltet sein, dass eine dritte Expansions-Charakteristik, die verschieden ist von der zweiten Expansions-Charakteristik, bereitgestellt ist. Alternativ können auch dritte Zirkumferenzsegmente vorgesehen sein, die eben jene dritte Expansions-Charakteristik definieren. Dies bewirkt eine mehrfach abgestufte Nachexpansion des Stents bei Erweiterung eines Gefäßes. Die unterschiedliche Stegstärke bzw. allgemein Radial-Steifigkeit führt zu unterschiedlichen Radialkräften und damit zu einem mehrfach abgestuften radialen Kräfteprofil des Stents. Beispielsweise haben die weichen Stentsegmente 20% bis 60%, vorzugsweise 20% bis 30% der Stegbreite der harten Stentsegmente. Die Zahl der zweiten Zirkumferenzsegmente (weichen Stentsegmente) innerhalb eines Ringsegments kann variieren und richtet sich nach der jeweils gewünschten Expansionsreserve.

Vorzugsweise besteht der erfindungsgemäße Stent aus einer Formgedächtnislegierung. Diese kann beispielsweise ein Federstahl sein, der für medizinische Zwecke zugelassen ist, ist aber insbesondere eine Nickel-Titan-Legierung, beispielsweise Nitinol. In diesem Fall ist der Stent selbstexpandierenden.

Die erfindungsgemäßen Stents können auf übliche Art und Weise durch Laserschneiden aus einem Rohr hergestellt werden. Grundsätzlich sind bei den weichen Stentsegmenten (zweiten Zirkumferenzsegmente) wenigstens zwei Varianten möglich. Zum einen bleiben die weichen Stentsegmente (zweiten Zirkumferenzsegmente) bei der Formgebung fixiert und komplett geschlossen. Bei einer Gefäßerweiterung über den Nominaldurchmesser des Stents hinaus werden diese weichen Stegsemente (zweiten Zirkumferenzsegmente) im Bereich der Expansionsreserve rein passiv (durch Zug des Gefäßes) geöffnet (F2 = 0).

Zum anderen werden die weichen Segmente (zweiten Zirkumferenzsegmente) bereits in der Formgebung ganz oder teilweise geöffnet. In diesem Fall entwickeln die weichen Segmente (zweiten Zirkumferenzsegmente) im Bereich der Expansionsreserve des Stents eine COF (F2>0), die es dem Stent erlaubt, über den Nominaldurchmesser im Bereich seiner Expansionsreserve aktiv mitzuwachsen. Der erfindungsgemäße Stent ist insbesondere auch für im Wachstum befindliche Gefäße bei Kindern geeignet. Es versteht sich, dass die harten und weichen Segmente (ersten und zweiten Zirkumferenzsegmente) nicht nur über die Stegstärke erzielt werden können, sondern auch über die Steglänge und über die Gestaltung und Größe der Zellen des Stents.

Es versteht sich ferner, dass die weichen Segmente (zweite Zirkumferenzsegmente) sowohl gleichmäßig als auch ungleichmäßig über die Länge des Stents verteilt sein können. Beispielsweise können die axialen Stentenden eine größere oder kleinere Expansionsreserve, vorzugsweise mehr oder weniger zweite Zirkumferenzsegmente oder anders gestaltete Zirkumferenzsegmente aufweisen als die Stentmitte.

In einer weiteren bevorzugten Ausführungsform weist der Stent große und kleine Zellen auf, wobei die großen Zellen zweite Zirkumferenzsegmente (weiche Stentsegmente) ausbilden und die kleinen Zellen erste Zirkumferenzsegmente (harte Stentsegmente). Die Begriffe "klein" und "groß" beziehen sich hier auf die Gestaltung der Zirkumferenzsegmente und sind nicht absolut zu verstehen. Große Zellen unterscheiden sich von kleinen Zellen insbesondere dadurch, dass sie längere Seiten haben als die kleinen Zellen und im expandierten Zustand eine größere Zellenfläche haben als kleine Zellen. Große und kleine Zellen können also auch über die unterschiedlichen Seitenlängen beschrieben werden, die in der Regel durch Stege gebildet werden. Sie können auch allgemein als erste und zweite Zellen bezeichnet werden. Ein längerer Steg hat im Allgemeinen eine geringere Steifigkeit (Verhältnis von Kraft / Verformung), als ein kurzer Steg. Dieser Ausführungsform liegt also die Erkenntnis zugrunde, dass unterschiedliche dauerhafte Auswärtskräfte (COF) bzw. Radialkraftniveaus nicht nur durch Variation des Querschnitts eines Stegs erreicht werden können, sondern auch durch Variation seiner freien Länge, womit eine größere Zelle erreicht wird. Die Zellen müssen aber nicht alle geschlossen sein, vielmehr sind auch offene Zellen, die beispielsweise nur an zwei oder drei Seiten begrenzt sind, umfasst. Ferner liegt dieser Ausführungsform die Erkenntnis zugrunde, dass axial benachbarte Stege analog zu parallel angeordneten bzw. parallel-geschalteten Federn wirken. Große Zellen führen im Vergleich zu kleinen Zellen zu einer geringeren Anzahl an nebeneinander angeordneten Stegen, somit zu weniger parallel-geschalteten Federelementen, wodurch die Radialkraft in diesen Abschnitten reduziert werden kann.

In einer bevorzugten Weiterbildung sind kleine Zellen in axialer Richtung des Stents aneinander grenzend aufgereiht, bestehen aus jeweils vier Stegen und sind über ihre Eckpunkte miteinander verbunden Große Zellen sind zwischen den Reihen aus kleinen Zellen angeordnet, wobei die großen Zellen von jeweils wenigstens sechs Stegen gebildet werden. Die kleinen Zellen definieren bei der Expansion des Stents den Nominaldurchmesser und die großen Zellen bilden eine Expansionsreserve.

Vorzugsweise sind die Ringsegmente so angeordnet, dass sie zwischen sich eine Vielzahl von kleinen und großen Zellen ausbilden. Dabei verlaufen die kleinen Zellen vorzugsweise aneinandergrenzend und aufgereiht in axialer Richtung und werden in einer Ausführungsform aus jeweils vier Stegen gebildet. In axialer Richtung aneinandergrenzende Zellen sind vorzugsweise an ihren Eckpunkten entweder über Verbindungsstege oder unmittelbar über gemeinsame Eckpunkte oder Knotenpunkte miteinander verbunden.

Zwischen den Ringsegmenten und zwischen den in axialer Richtung verlaufenden kleinen Zellen sind große Zellen angeordnet, die in einer Ausführungsform von jeweils wenigstens sechs Stegen gebildet werden. Zweckmäßig sind auch aus acht oder mehr Stegen gebildete große Zellen.

Die kleinen Zellen bilden eine Expansionsmatrix aus, die bei der Expansion den Nominaldurchmesser definieren. Die kleinen Zellen stellen aufgrund ihrer geringen Stegzahl/Fläche eine große Radialkraft bereit, die für eine Gefäßaufweitung maßgeblich ist. Die großen Zellen bilden eine Expansionsreserve und definieren den Maximaldurchmesser, der durch aktive oder passive Nachexpansion einem sich erweiternden Gefäß folgen kann. Aufgrund der größeren Zahl der Stege/Fläche stellen diese großen Zellen eine geringere Radialkraft bereit, was sich insbesondere für die passive Nachexpansion, bei der der Stent dem sich erweiternden Gefäß folgen soll, als Vorteil erweist.

Es versteht sich, dass kleine Zellen auch zwischen den Ringsegmenten angeordnet sein können und beispielsweise zwei benachbarte Ringsegmente über ihre Eckpunkte miteinander verbinden.

Die kleinen Zellen stellen somit die harten Stentsegmente (erste Zirkumferenzsegmente) mit hoher Radialkraft dar, die großen Zellen die weichen Stentsegmente (zweite Zirkumferenzsegmente) mit geringerer Radialkraft.

Die großen Zellen sind vorzugsweise jeweils zwischen den in axialen Reihen angeordneten kleinen Zellen angeordnet, wobei vorzugsweise zwei große Zellen zwischen zwei Ringsegmenten angeordnet sind. In axialer Richtung können die großen Zellen linear, vorzugsweise axial benachbart und fluchtend oder aber spiralförmig gegeneinander versetzt angeordnet sein.

Durch die Formgebung, insbesondere durch eine teilweise Vorexpansion, ist es möglich, auch den großen Zellen (weichen Stentsegmente) ein aktives Expansionsverhalten mit einer - im Vergleich zu den kleinen Zellen - geringeren Radialkraft zu verleihen. Dies bedeutet, dass im Falle einer Gefäßerweiterung auch die großen Zellen dieser Erweiterung aktiv mit einer eigenen, geringen COF folgen können.

Die Erfindung betrifft ferner die Verwendung von Stents nach einer der vorstehend beschriebenen bevorzugten Ausführungsbeispiele zur Variation der Radialkraft zur Erzeugung eines gestuften Radialkraftprofils. Ferner betrifft die Erfindung die Verwendung, dass die harten und weichen Stentsegmente durch Stege unterschiedlicher Länge und/oder Stärke oder durch Zellen unterschiedlicher Größe gebildet werden.

In einem weiteren Aspekt wird die eingangs genannte Aufgabe gelöst durch ein Herstellungsverfahren für einen Stent, vorzugsweise einen Stent nach einer der vorstehend beschriebenen bevorzugten Ausführungsformen eines Stents nach dem ersten Aspekt der Erfindung, mit den Schritten: Bereitstellen eines Rohrstücks aus einem Formgedächtnismaterial; Schneiden des Stents aus dem Rohrstück, wobei der Stent erste und zweite Zirkumferenzsegmente aufweist; mechanisches Verschließen der zweiten Zirkumferenzsegmente; Durchführen eines ersten Formgebungsschritts durch mechanisches Aufdehnen der ersten Zirkumferenzsegmente; im Anschluss hieran: Lösen des mechanischen Verschlusses der zweiten Zirkumferenzsegmente und Durchführen eines zweiten Formgebungsschritts durch mechanisches Aufdehnen der zweiten Zirkumferenzsegmente. Dem Verfahren liegt die Erkenntnis zugrunde, dass die zweiten Zirkumferenzsegmente eine geringere Radialkraft entwickeln als die ersten Zirkumferenzsegmente. Beim Aufdehnen des Stents, um ihn aus dem zunächst geschnittenen Zustand in die expandierte Form zu bringen, ist es daher zweckdienlich, zunächst mit einer hohen Kraft die ersten Zirkumferenzsegmente in Form zu bringen, dabei aber die zweiten Zirkumferenzsegmente zu verschie-ßen, sodass diese nicht überlastet werden. Dann kann in einem zweiten Schritt der Verschluss der zweiten Zirkumferenzsegmente gelöst und diese mit einer geringeren Kraft gedehnt werden, um sie in Form zu bringen. Alternativ wäre es auch möglich, zunächst ohne Verschluss mit einer geringen Kraft zu dehnen, sodass nur die zweiten Zirkumferenzsegmente in Form gebracht werden, diese anschließend zu verschließen (entweder im expandierten Zustand oder zu diesem Zweck in einem teilgecrimpten Zustand) und dann mit höherer Kraft die ersten Zirkumferenzsegmente zu dehnen. In beiden Alternativen werden aber die zweiten Zirkumferenzsegmente beim Dehnen der ersten Zirkumferenzsegmente verschlossen und so aus dem Kraftfluss genommen, sodass die Kraft beim Dehnen der ersten Zirkumferenzsegmente ausschließlich auf diese wirkt.

Das mechanisches Verschließen der zweiten Zirkumferenzsegmente umfasst vorzugsweise: Einsetzen eines mechanischen Haltemittels, insbesondere einer Klammer, zum mechanischen Verschließen der zweiten Zirkumferenz-segmente. Alternativ bevorzugt umfasst das mechanisches Verschließen der zweiten Zirkumferenzsegmente: Einbringen von Materialbrücken zwischen Stege der zweiten Zirkumferenzsegmente, vorzugsweise während des Schritts des Schneidens. Auch andere Verschlusstechniken sind denkbar und bevorzugt, wie beispielsweise temporär angebrachte Haltebänder.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsformen sowie anhand der Zeichnungen; diese zeigen in:
Figur 1 den Endbereich eines erfindungsgemäßen Stents in flächige Darstellung,
Figur 2 schematisch ein Ringsegment eines erfindungsgemäßen Stents in expandierten Zustand,
Figur 3 schematisch ein Ringsegment eines erfindungsgemäßen Stents in nachexpandiertem Zustand (Expansionsreserve),
Figur 4 ein Diagramm, das den Zusammenhang zwischen Radialkraft und Aufweitung/Durchmesser eines Stents illustriert;
Figur 5 ein weiteres Diagramm, das den Zusammenhang zwischen Radialkraft und Aufweitung/Durchmesser des Stents illustriert;
Figuren 6a bis 6c ein Ringsegment des Stents in einem gecrimpten Zustand, bei Nominaldurchmesser und bei Maximaldurchmesser;
Figuren 7a bis 7c ein Ringsegment des Stents in einem weiteren Ausführungsbeispiel in einem gecrimpten Zustand, bei Nominaldurchmesser und bei Maximaldurchmesser;
Figuren 8a bis 8c ein Ringsegment des Stents in einem weiteren Ausführungsbeispiel in einem gecrimpten Zustand, bei Nominaldurchmesser und bei Maximaldurchmesser;
Figur 9 ein weiteres Ausführungsbeispiel eines Stents in flächiger Darstellung; und in
Figur 10 ein weiteres Ausführungsbeispiel eines Stents in flächiger Darstellung.

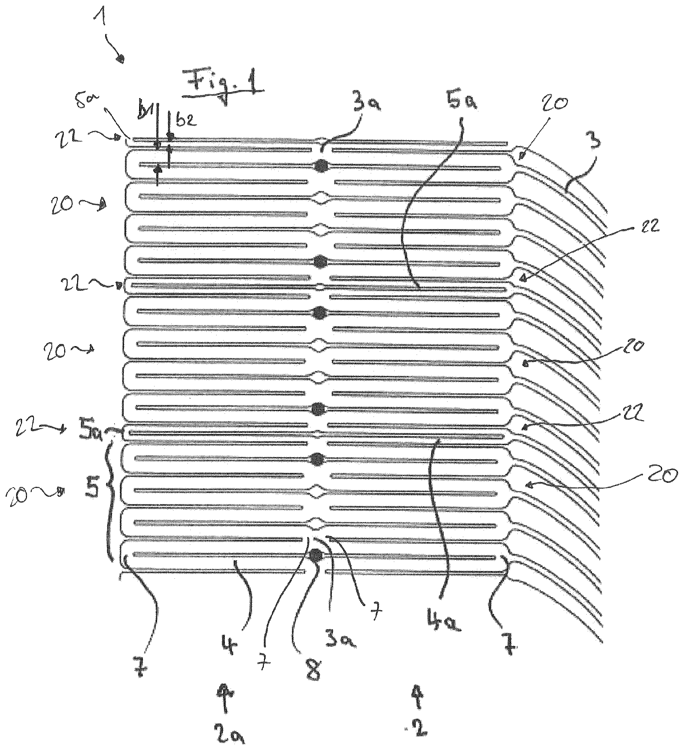

Figur 1 zeigt den Endbereich eines nicht expandierten erfindungsgemäßen Stents 1 (selbstexpandierend) in flächiger Darstellung. Es versteht sich, dass der Stent 1 im Allgemeinen eine Rohrstruktur hat, hier aber zur besseren Veranschaulichung flächig, d.h. nur die Oberfläche des ansonsten rohrförmigen Stents dargestellt ist.

In der in Figur 1 gezeigten Ausführungsform hat der Stent 1 zwei Ringsegmente 2, 2a, die axial (mit Bezug auf Fig. 1 horizontal) nebeneinander und benachbart zueinander angeordnet sind. Die einzelnen Ringsegmente 2 und 2a sind mit ihren benachbarten Ringsegmenten 2 und 2a verbunden, hier durch Verbindungsstege 3. Das heißt, mit Bezug auf Figur 1 rechts neben dem Ringsegment 2 können sich ein oder weitere Ringsegmente (nicht gezeigt) anschließen, die mit dem Ringsegment 2 über die Verbindungsstege 3 verbunden sind. Die Verbindungsstege 3 sichern vor allem die axiale Ausrichtung der einzelnen Ringsegmente 2, 2a, bewirken aber selbst keine oder keine wesentliche Radialkraft. Im dargestellten Stentabschnitt ist das randständige Ringsegment 2a ferner mit dem benachbarten Ringsegment 2 unmittelbar an Kontaktstellen 3a der Bögen 7 verbunden, die sich axial benachbart gegenüberliegen. Die einzelnen Stege 4, 4a der Ringsegmente 2 und 2a mäandrieren, sodass sie sich bei der Expansion zickzack-förmig aufspreizen. Alle Stege 4 und 4a sind durch Bögen 7 mit ihren Nachbarstegen 4 und 4a verbunden.

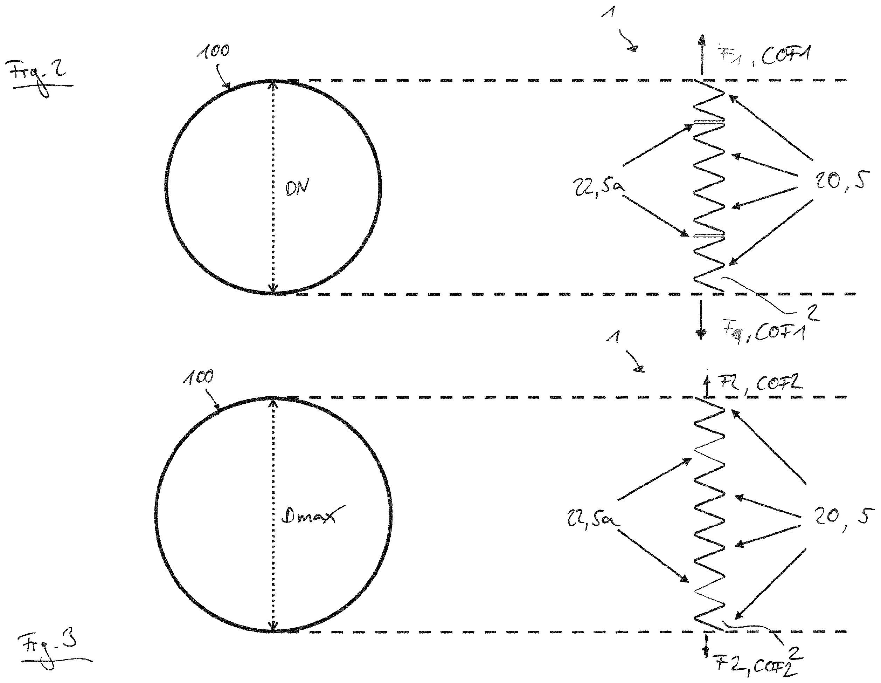

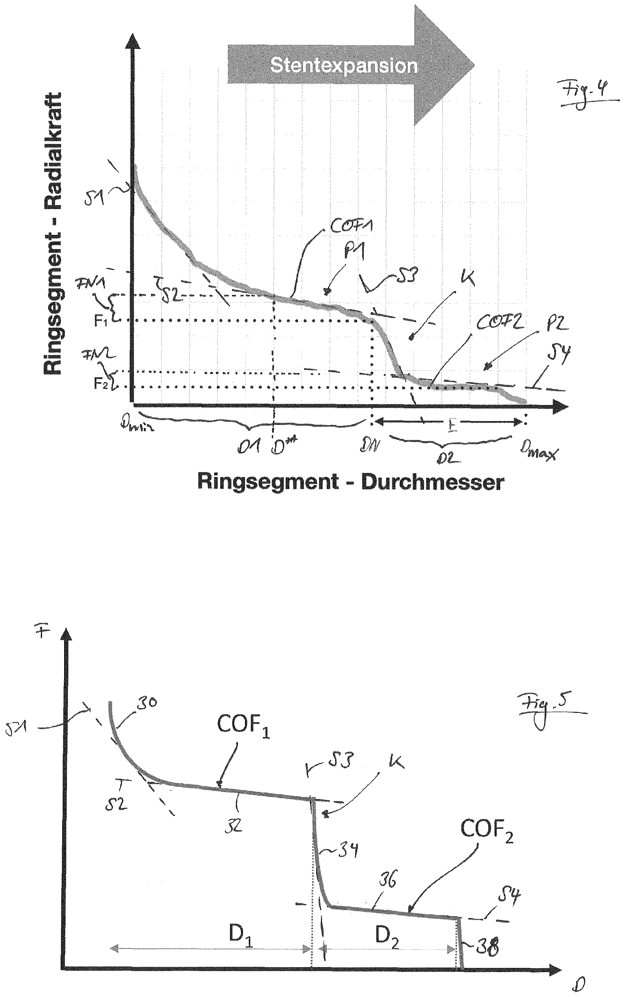

Der Stent 1 ist so gebildet, dass die Ringsegmente 2, 2a in einem ersten Durchmesserbereich D1 (vgl. Fig. 4, 5) kleiner als der Nominaldurchmesser DN eine erste Expansions-Charakteristik aufweisen, und in einem zweiten Durchmesserbereich D2 größer als der Nominaldurchmesser DN eine zweite Expansions-Charakteristik aufweisen, wobei sich die erste und die zweite Expansions-Charakteristik unterscheiden. Auf diese Weise ist der Stent 1 dazu in der Lage, im ersten Durchmesserbereich D1 eine erste dauerhafte Auswärtskraft (chronic outward force) COF1, vorzugsweise eine erste Radialkraft in einem ersten Bereich, vorzugsweise, und in dem zweiten Durchmesserbereich D2 eine zweite dauerhafte Auswärtskraft (chronic outward force) COF2, vorzugsweise eine zweite Radialkraft in einem zweiten Bereich, bereitstellen. Dies wird im Folgenden und auch mit Bezug auf die Figuren 2-5 noch genauer beschrieben werden.

In dem ersten, in Figur 1 gezeigten Ausführungsbeispiel hat jedes der Ringsegmente 2, 2a mehrere Zirkumferenzsegmente, hier insgesamt drei erste Zirkumferenzsegmente 20 und drei zweite Zirkumferenzsegmente 22, die jeweils um den Umfang des jeweiligen Ringsegments 2, 2a abwechselnd angeordnet sind. In diesem Sinn sind die zweiten Zirkumferenzsegmente 22 gleichmäßig um den Umfang verteilt, in diesem Fall jeweils um 120° versetzt zueinander. Eine gleichmäßige Verteilung ist vorteilhaft, um ein gleichmäßiges Expandieren des Stents 1 zu erzielen. Es können auch mehr oder weniger als jeweils drei erste und drei zweite Zirkumferenzsegmente 20, 22 vorgesehen sein. Insbesondere kann ein Stent 1 mit nur einem zweiten Zirkumferenzsegment 22 ebenso erfindungsgemäß sein. In weiteren Ausführungsformen können auch ein oder mehrere dritte Zirkumferenzsegmente (nicht gezeigt) vorgesehen sein, um eine weitere Abstufung der Radialkraft zu erzielen.

Im in Figur 1 dargestellten Stent 1 sind in jedem Ringsegment 2 und 2a jeweils sechs Stege 4a geringerer Breite b2 angeordnet. Diese Stege 4a geringerer Breite b2 bilden paarweise die zweiten Zirkumferenzsegmente 22, auch "weiche Stentsegmente 5a" genannt, aus. Die ersten Zirkumferenzsegmente 20, auch "harte Stentsegmente 5" genannt, weisen in dem hier gezeigten Ausführungsbeispiel jeweils acht Stege 4 bzw. vier Stegpaare auf. Die Stege 4 der ersten Zirkumferenzsegmente 20 haben eine erste Breite b2. Bei der normalen Expansion von einem gecrimpten Zustand auf Nominaldurchmesser werden nur die ersten Zirkumferenzsegmente 20 (harten Stentsegmente 5) aufgespreizt, während die zweiten Zirkumferenzsegmente 22 (weichen Stentsegmente 5a) in ihrem geschlossenen Zustand verbleiben.

Im dargestellten Fall sind die Stege 4a der zweiten Zirkumferenzsegmente 22 (weichen Stentsegmente 5a) in etwa halb so breit wie die Stege 4 der ersten Zirkumferenzsegmente 20 (harten Stentsegmente 5). Die Kraft, die nötig ist, um die zweiten Zirkumferenzsegmente 22 (weichen Stentsegmente 5a) aufzuspreizen ist entsprechend geringer und kann ohne weiteres von einem sich aufweitenden Gefäß aufgebracht werden (passive Expansion). Alternativ können die zweiten Zirkumferenzsegmente 22 (weichen Stentsegmente) über die Formgebung eine Expansionsreserve haben, die die aktive Expansion bei sich aufweitendem Gefäß zu ermöglichen. Die Dicke in radialer Richtung sowie die Länge in axialer Richtung gemessen der einzelnen Stege 4, 4a ist in diesem Ausführungsbeispiel bei allen Stegen 4, 4a gleich; der Stent 1 ist vorzugsweise aus einem einheitlichen Rohrmaterial geschnitten. Die erste Breite b1 der ersten Stege 4 der ersten Zirkumferenzsegmente 20 definiert vorzugsweise eine erste Radial-Steifigkeit, die zweite Breite b2 der zweiten Stege 4a der zweiten Zirkumferenzsegmente 22 definiert vorzugsweise eine zweite Radial-Steifigkeit. Die Radial-Steifigkeit und auch ein Radialkraft-Diameter-Profil ist in dem Ausführungsbeispiel der Figur 1 demnach durch die Form und Gestaltung der Stege 4, 4a vorgegeben und kann durch diese eingestellt werden. Vorzugsweise ist die erste Breite b1 der ersten Stege 4 der ersten Zirkumferenzsegmente 20 um einen Faktor größer als die zweite Breite b2 der zweiten Stege 4a der zweiten Zirkumferenzsegmente 22. Ein Verhältnis b1/b2 ist Vorzugsweise in einem Bereich von 1,5 bis 5, vorzugsweise 1,5 bis 4, weiter bevorzugt 2 bis 3,5.

Die Bögen 7 sind die Ansatzstellen der Verbindungsstege 3 und die Dreh- bzw. Biegepunkte bei der Aufspreizung bzw. beim Crimpen eines selbstexpandierenden Stents 1.

Die dargestellten Punkte 8 sind die Fixierstellen, an denen der Stent 1 bei der Formgebung so an einem Träger fixiert wird, dass die weichen Stentsegmente 5a im Nominaldurchmesser geschlossen bleiben.

An dem dargestellten rechten Endbereich kann sich ein nicht dargestellter zentraler Bereich des Stents 1 über die Verbindungsstege 3 anschließen, der, bis auf die weichen Stentsegmente 5a, ein konventionelles Design aufweisen kann. Der Stent 1 kann aber auch nur aus den zwei Ringsegmenten 2, 2a gebildet sein und insofern keine Verbindungsstege 3 aufweisen. Auch ist es denkbar, dass statt der Verbindungsstege 3 unmittelbar über Kontaktstellen 3a an dem mit Bezug auf Fig. 1 rechten axialen Ende ein oder mehrere weitere Ringsegmente angeschlossen sind, die identisch oder ähnlich zu den Ringsegmenten 2, 2a gebildet sein können.

Figur 2 zeigt schematisch einen Stent 1 mit seinem Nominaldurchmesser DN nach der Implantation in einem Gefäß 100. Das dargestellte Ringsegment 2 zeigt die aufgespreizten ersten Zirkumferenzsegmente 20 (harten Stentsegmente 5) in offenen Zustand, während die zweiten Zirkumferenzsegmente 22 (weichen Stentsegmente 5a) noch geschlossen sind. F1 bezeichnet die hohen Radialkräfte der ersten Zirkumferenzsegmente 20 (harten Stentsegmente 5).

Im Vergleich zu dem Ausführungsbeispiel der Figur 1 ist in Figur 2 und 3 jeweils nur ein Ringsegment 2 gezeigt. Es soll aber verstanden werden, dass das Ausführungsbeispiel der Figur 2 und 3 ebenso zwei oder mehr Ringsegmente 2, 2a umfassen kann. Insofern dient die Darstellung mit nur einem Ringsegment 2 in den Figuren 2, 3 insbesondere illustrativen Zwecken. Während Figur 1 den gecrimpten Zustand des Stents 1 zeigt, in dem dieser auf einen minimalen Durchmesser Dmin (vgl. auch Fig. 4-6) komprimiert ist, um bei diesem minimalen Durchmesser Dmin im gecrimpten Zustand implantiert zu werden. Im gecrimpten Zustand (vgl. Figur 1) sind die einzelnen Stege 4, 4a im Wesentlichen parallel zueinander. Im expandierten Zustand sind die Stege 4, 4a winklig zueinander in spannen einen Raum oder Zellen zwischen sich auf.

Bei der Expansion auf den Nominaldurchmesser DN (Figur 2) sind nur die ersten Zirkumferenzsegmente 20 vollständig expandiert, während die zweiten Zirkumferenzsegmente 22 noch geschlossen bzw. in einem teilweise gecrimpten Zustand sind. Im vollständig expandierten Zustand der ersten Zirkumferenzsegmente 20 üben die ersten Zirkumferenzsegmente 20 keine weitere Radialkraft mehr aus. Eine Radial-Steifigkeit der zweiten Zirkumferenzsegmente ist vorzugsweise so gewählt, dass eine zweite Radialkraft F2, die durch die zweiten Zirkumferenzsegmente 22 bewirkt werden kann wesentlich geringer ist als eine erste Radialkraft F1, die durch die ersten Zirkumferenzsegmente 20 bewirkt wird. Zudem ist die zweite Radial-Steifigkeit der zweiten Zirkumferenzsegmente 22 so gewählt, dass die von diesen bewirkte zweite Radialkraft F2 so gering ist, dass eine Aufweitung des Gefäßes 100 nicht stattfindet. Insofern werden die zweiten Zirkumferenzsegmente 22 bis zum Nominaldurchmesser DN von den ersten Zirkumferenzsegmente 20 geschlossen gehalten; die zweiten Zirkumferenzsegmente 22 können nicht gegen die von den ersten Zirkumferenzsegmenten 20 entwickelte erste Radialkraft F1 expandieren, da die von diesen entwickelte zweite Radialkraft F2 zu gering ist.

Figur 3 zeigt nun schematisch den Stent 1 mit seinen Maximaldurchmesser Dmax nach Erweiterung des Gefäßes 100 und vollständiger Expansion der ersten Zirkumferenzsegmente 20. Das dargestellte Ringsegment 2 zeigt die aufgespreizten ersten Zirkumferenzsegmente 20 (harten Stentsegmente 5) in offenem Zustand, wie in Figur 2 dargestellt, und die ebenfalls aufgespreizten zweiten Zirkumferenzsegmente 22 (weichen Stentsegmente 5a), die der Gefäßerweiterung gefolgt sind. F2 bezeichnet die für die Nachexpansion verantwortlichen geringeren Radialkräfte der zweiten Zirkumferenzsegmente 22 (weichen Stentsegmente).

Nachdem der Stent 1 durch die von den ersten Zirkumferenzsegmenten 20 und zweiten Zirkumferenzsegmente 22 entwickelte Radialkraft auf den Nominaldurchmesser DN expandiert ist und dabei ggf. das Gefäß 100 im Durchmesser etwas aufgeweitet hat unter Aufbringen einer ersten dauerhaften Auswärtskraft (chronic outward force) COF1, kann es immer noch zu einem weiteren Ausweichen der Gefäßwand und letztlich zum weiteren Aufweiten des Gefäßes 100 kommen (sog. Remodeling). Der Stent 1 ermöglicht es, in einem Durchmesserbereich D2 größer als der Nominaldurchmesser DN immer noch eine Kraft aufzubringen, nämlich eine zweite dauerhafte Auswärtskraft (Radialkraft) COF2. In dem in den Figuren 1 bis 3 gezeigten Ausführungsbeispiel ist das über die zweiten Zirkumferenzsegmente 22 realisiert, die nur eine derartige Steifigkeit (zweite Radial-Steifigkeit) aufweisen, dass sie die zweite Radialkraft F2 entwickeln. Diese ist so gering gewählt, dass ein weiteres Aufweiten oder Remodeling des Gefäßes 100 verhindert wird. Die zweite Radialkraft F2 ist vorzugsweise in einem Bereich gewählt, der lediglich ein Folgen der Gefäßwand durch den Stent 1 ermöglicht.

Beim Maximaldurchmesser Dmax sind auch die zweiten Zirkumferenzsegmente 22 vollständig expandiert. Für den Fall, dass die zweiten Zirkumferenzsegmente 22 derart ausgelegt sind, dass sie eine Radialkraft F2 = 0 entwickeln, ist der Maximaldurchmesser insbesondere durch den Durchmesser definiert, bei dessen Überschreitung eine Radialkraft nach innen wirkt, d.h. das Gefäß 100 beim weiteren Remodeling an dem Stent 1 ziehen müsste. Für den Fall, dass die zweiten Zirkumferenzsegmente 22 derart ausgelegt sind, dass sie eine Radialkraft F2 > 0 entwickeln, ist der Maximaldurchmesser insbesondere durch den Durchmesser definiert, bei dem der Stent 1 in einer entspannten Stellung ist. Hier würde bei dessen Überschreitung wiederum eine Radialkraft nach innen wirken, d.h. das Gefäß 100 beim weiteren Remodeling an dem Stent 1 ziehen müsste. Allerdings ist die zweite Radial-Steifigkeit der zweiten Zirkumferenzsegmente 22 vorzugsweise derart gewählt, dass keine Beeinflussung der Gefäßwand sei des durch eine wesentliche Radialkraft nach außen oder eine wesentliche Radialkraft nach innen, herrscht. Ein Vergrößern des Gefäßes 100 über den Nominaldurchmesser DN ist demnach möglich, auch ohne Lockerung des Stents 1, ein Zusammenziehen des Gefäßes 100 kleiner als der Nominaldurchmesser DN wird wirksam vermieden.

Figur 4 zeigt den Radialkraftverlauf eines Stents 1 gemäß Figur 1 in Abhängigkeit vom Aufweitungsgrad bzw. Durchmesser. Nach der Implantation weitet sich der Stent 1, der selbstexpandierend ausgebildet ist, zum Nominaldurchmesser DN auf, bei abfallender Radialkraft und übt eine erste Radialkraft F1 in einem ersten Bereich aus. Der Nominaldurchmesser ist bei DN erreicht. Ausschließend wird beim Remodeling des Gefäßes 100 der Maximaldurchmesser Dmax erreicht. Bei dieser Nachexpansion wirkt die verminderte zweite Radialkraft F2 in einem zweiten Radialkraftbereich. Der Bereich E bezeichnet die Expansionsreserve.

Die erste dauerhafte Auswärtskraft COF1 ist hier definiert durch den Bereich, in welchem der Stent 1 beim Expandieren mit dem Gefäß 100 in Kontakt kommen wird bzw. kann. In Fig. 4 wird bei dem Nominaldurchmesser DN genau eine Kraft F1 ausgeübt. Ist das Gefäß 100 aber etwas kleiner als der Nominaldurchmesser DN, wird eine leicht höhere Radialkraft ausgeübt. Der Stent 1 sollte so ausgelegt sein, dass er mit dem Gefäß in einem Bereich in Kontakt kommt, in welchem die erste dauerhafte Auswärtskraft COF1 bei einem ersten Plateau P1 ist, also zwischen dem Durchmesser D* und dem Nominaldurchmesser DN. In diesem Bereich ist die erste dauerhafte Auswärtskraft COF1 in einem ersten Radialkraftniveau FN1.

Von dem minimalen Durchmesser Dmin im gecrimpten Zustand des Stents 1 fällt die ausgeübte Radialkraft rasch ab bis sie etwa nach Hälfte des ersten Durchmesserbereichs D1 (bei D*) zwischen dem minimalen Durchmesser Dmin und dem Nominaldurchmesser DN, eine Höhe im Bereich eines ersten Radialkraftniveaus FN1 erreicht, welches annäherungsweise als konstant aufgefasst werden kann. Bis hierhin fällt die Radialkraft degressiv ab. Der Stent 1 hat bis hierhin eine erste Expansions-Charakteristik, nämlich eine degressive. Das Gefäß 100 wird durch die Kraft des Stents 1 radial bis zum Nominaldurchmesser DN aufgedehnt. Aus Figur 4 ist ersichtlich, dass die Radialkraft ab dem Nominaldurchmesser DN stufenförmig abfällt in den Bereich eines zweiten Radialkraftniveaus FN2, und insbesondere auf die zweite Radialkraft F2, die im Bereich des zweiten Radialkraftniveaus FN2 wiederum als im Wesentlichen konstant aufgefasst werden kann. Der Stent 1 kann also eine zweite dauerhafte Auswärtskraft COF2 ausüben. In dem zweiten Durchmesserbereich D2 fällt die Radialkraft damit erneut degressiv ab und der Stent 1 hat demnach eine zweite Expansions-Charakteristik. Diese ist von der ersten verschieden, da der Grad des Abfalls und das erreichte Kraftniveau verschieden ist. Die bereitgestellte zweite dauerhafte Auswärtskraft COF2 ist derart gering, dass das Gefäß 100 nicht weiter aktiv durch den Stent 1 aufgedehnt wird, sondern der Stent 1 einem etwaigen Remodeling des Gefäßes 100 sanft und ohne wesentliche Kraft lediglich folgt. Der zweite Durchmesserbereich D2, größer als der Nominaldurchmesser DN stellt somit eine Expansionsreserve E dar. Wie ebenfalls aus Figur 4 zu erkennen ist, weist der Radialkraft-Durchmesser-Verlauf im Bereich des Nominaldurchmessers DN einen Knick K1 bzw. eine Stufe auf. In diesem Bereich verläuft die Kurve nicht mehr stetig und asymptotisch, wie von herkömmlichen Stents bekannt, sondern fällt plötzlich ab auf das zweite Radialkraftniveau FN2. Die erste Radialkraft F1 beträgt in dem gezeigten Ausführungsbeispiel der Figur 4 etwa das 5-fache bis 6-fache der zweiten Radialkraft F2.

Ferner sind in Figur 4 Steigungen S1 bis S4 als Geraden eingezeichnet. In dem ersten Durchmesserbereich D1 nähert sich die Radialkraft asymptotisch der Geraden S2 an, die eine zweite Steigung S2 angibt. In dem zweiten Durchmesserbereich D2 nähert sich die Radialkraft asymptotisch der Geraden S4 an, die eine vierte Steigung S4 angibt. Sowohl die zweite als auch die vierte Steigung S2, S4 sind gering und können im Wesentlichen als linear aufgefasst werden. Ausgehend vom gecrimpten Zustand bei Dmin fällt die Radialkraft zunächst stark mit einer Steigung S1, die hier tangential angetragen ist, ab, um dann von dem Durchmesser D* bis zum Nominaldurchmesser DN mit der zweiten Steigung S2 abzufallen. Die erste Steigung S1 ist deutlich größer als die zweite Steigung S2. Bei einer Expansion über den Nominaldurchmesser DN hinaus fällt die Radialkraft dann wieder zunächst mit einer größeren Steigung S3 ab, um dann wiederum mit einer geringeren Steigung S4 abzufallen.

In Figur 5 zeigt einen weiteren Radialkraft-Diameter-Verlauf des erfindungsgemäßen Stents 1. Wiederum vom minimalen Durchmesser Dmin im gecrimpten Zustand des Stents 1 fällt die Radialkraft, auf der Ordinate aufgetragen, zunächst in einem ersten Abschnitt 30 degressiv ab, bis die Radialkraft ein erstes Plateau 32 in dem ersten Durchmesserbereich D1 erreicht. Auf diesem ersten Plateau 32, das in dem Ausführungsbeispiel der Fig. 4 dem ersten Radialkraftniveau FN1 entspricht, muss nicht zwingend vollständig über den ersten Durchmesserbereich D1 eine konstante Kraft ausgeübt werden, auch wenn dies wünschenswert ist. Die in diesem Bereich erzeugte erste dauerhafte Auswärtskraft COF1 bleibt beim Expandieren bis zum Nominaldurchmesser DN in etwa konstant, fällt mit der Steigung S2 leicht ab. Beim Expandieren des Stents 1 über den Nominaldurchmesser DN hinaus fällt die Radialkraft dann abrupt in einer Stufe 34 mit der dritten Steigung S3 ab, die nicht zwingend linear sein muss, sondern auch degressiv verlaufen kann. Bevorzugt ist, dass der Kraftabfall möglichst steil ist und die Radialkraft von dem Niveau der ersten dauerhaften Auswärtskraft COF1 möglichst unmittelbar bei Überschreiten des Nominaldurchmessers DN auf ein wesentlich niedrigeres Niveau abfällt. In dem in Figur 5 gezeigten Ausführungsbeispiel fällt die Radialkraft auf ein zweites Plateau 36 auf, welches hier analog zum ersten Plateau 32 wieder eine im Wesentlichen konstante Radialkraft repräsentiert, die hier als zweite dauerhafte Auswärtskraft COF2 bezeichnet ist. Das Niveau der zweiten dauerhaften Auswärtskraft COF2 bleibt über den zweiten Durchmesserbereich D2 im Wesentlichen konstant, fällt leicht mit der vierten Steigung S4 ab. Bei Erreichen des Maximaldurchmessers Dmax fällt die Radialkraft dann auf 0 ab. Dies erfolgt in dem hier gezeigten Ausführungsbeispiel wiederum in einer Stufe 38, könnte aber auch flacher auslaufen, linear oder degressiv. Die erste dauerhafte Auswärtskraft COF1 beträgt in dem in Figur 5 gezeigten Beispiel das 3- bis 3,5-fache der zweiten dauerhafte Auswärtskraft COF2.

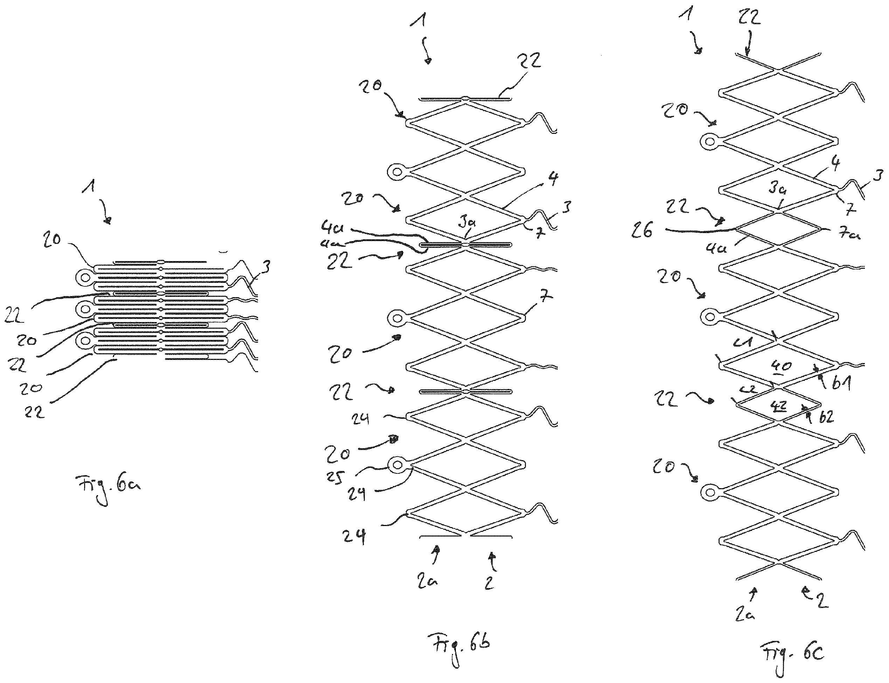

Die Figuren 6a bis 6c illustrieren ein weiteres Ausführungsbeispiel eines Stents 1. Alle drei Figuren zeigen den Umfang des Stents 1 in einer abgewickelten Darstellung. Es soll aber verstanden werden, dass der Stent 1 tatsächlich ringförmig ist und so das in den Figuren 6a bis 6c jeweils oben gezeigte Ende mit dem jeweils unten gezeigten Ende verbunden ist. Die Darstellungen kann auch als Schnittmuster für ein Laserschneidverfahren verstanden werden. In Figur 6a ist der Stents 1 im gecrimpten Zustand bei Minimaldurchmesser Dmin gezeigt, wiedergegeben. Figur 6a zeigt den Stent 1 dann bei Nominaldurchmesser DN und Figur 6c bei Maximaldurchmesser Dmax, also im voll expandierten Zustand.

Ebenso wie beim ersten Ausführungsbeispiel der Figur 1 hat der Stent 1 gemäß den Figuren 6a bis 6c je drei erste Zirkumferenzsegmente 20 und drei zweite Zirkumferenzsegmente 22. Die zweiten Zirkumferenzsegmente 22 haben jeweils wiederum je Ringsegment 2, 2a zwei zweite Stege 4a, die über einen zweiten Bogen 7a miteinander verbunden sind. Die ersten Zirkumferenzsegmente 20 haben je Ringsegment 2, 2a sechs erste Stege 4.

Die sechs ersten Stege 4 bilden in jedem ersten Zirkumferenzsegment 20 drei erste Zacken 24 (siehe Fig. 6b), von denen die mittlere Zacke optional mit einer Verankerung 25 versehen ist, hier als Ring ausgebildet. Die zweiten Stege 4a bilden gemeinsam je eine zweite Zacke 26. Die erste Breite b1 der ersten Stege 4 ist wiederum deutlich größer als die zweite Breite b2 der zweiten Stege 4a, hier etwa um den Faktor 3.

Im Unterschied zum ersten Ausführungsbeispiel der Figur 1 haben die zweiten Stege 4a der zweiten Zirkumferenzsegmente 22 eine andere Länge als die ersten Stege 4 der ersten Zirkumferenzsegmente 20. Konkret haben die zweiten Stege 4a eine zweite Länge L2, während die ersten Stege 4 eine erste Länge L1 haben. Die zweite Länge L2 ist in diesem Ausführungsbeispiel (Fig. 6a bis 6c) kürzer als die erste Länge L1. Dies kann die Steifigkeit leicht erhöhen, aber den Maximaldurchmesser Dmax begrenzen, was auch vorteilhaft sein kann, um ein übermäßiges Remodeling zu verhindern.

Die ersten Stege 4 der benachbarten Ringsegmente 2, 2a sind jeweils gegensinnig angeordnet, sodass sie erste Zellen 40 bilden. In analoger Weise bilden die zweiten Stege 4a der benachbarten Ringsegmente 2, 2a zweite Zellen 42 aus. Da die zweiten Stege 4a hier kürzer sind als die ersten Stege 4, sind die zweiten Zellen 42 kleiner als die ersten Zellen 40, hier etwa um 1/3.

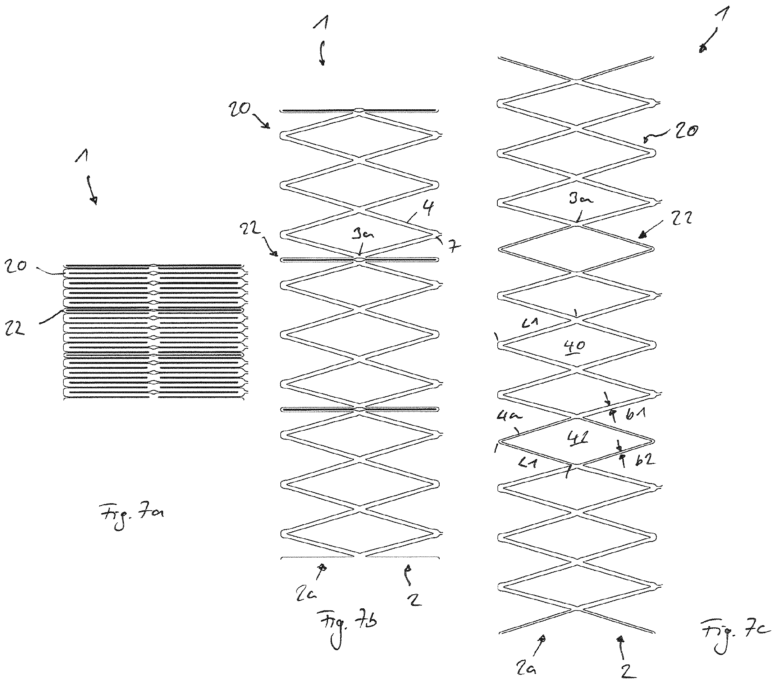

Die Figuren 7a bis 7c illustrieren in derselben Weise wie die Figuren 6a bis 6c den Stent 1 in einem weiteren Ausführungsbeispiel. Gleiche und ähnliche Elemente sind mit denselben Bezugszeichen wie in den Figuren 6a bis 6c benannt und im Folgenden werden im Wesentlichen die Unterschiede zum Ausführungsbeispiel der Figuren 6a bis 6c beschrieben.

In dem Ausführungsbeispiel gemäß Figur 7a bis 7c haben alle Stege, die ersten Stege 4 und die zweiten Stege 4a dieselbe Länge, hier L1. Die unterschiedlichen Steifigkeiten und damit auch Radialkräfte werden in diesem Ausführungsbeispiel ausschließlich über die Breite der jeweiligen Stege abgebildet. Die ersten Stege 4 haben eine erste Breite b1 und die zweiten Stege 4a haben eine zweite Breite b2. Da alle Stege dieselbe Länge haben, sind auch die von den Stegen gebildeten ersten und zweiten Zellen 40, 42 im Wesentlichen identisch.

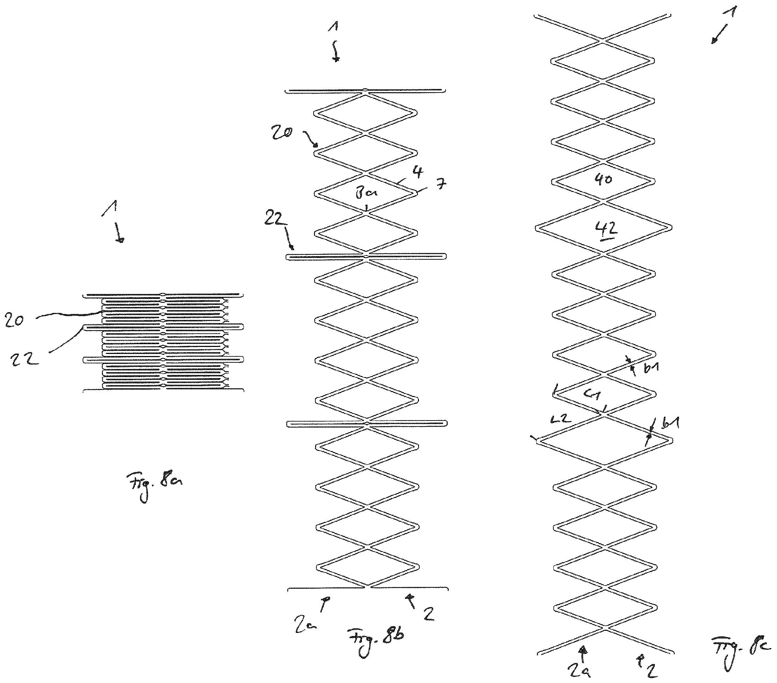

Die Figuren 8a bis 8c illustrieren in derselben Weise wie die Figuren 6a bis 6c den Stent 1 in einem weiteren Ausführungsbeispiel. Gleiche und ähnliche Elemente sind mit denselben Bezugszeichen wie in den Figuren 6a bis 6c benannt und im Folgenden werden im Wesentlichen die Unterschiede zum Ausführungsbeispiel der Figuren 6a bis 6c beschrieben.

Im Unterschied zum Ausführungsbeispiel der Figuren 6a bis 6c haben alle Stege, die ersten Stege 4 und die zweiten Stege 4a in dem Ausführungsbeispiel der Figuren 8a bis 8c dieselbe Stegbreite, hier b1. Die unterschiedliche Steifigkeit bzw. damit einhergehend die Radialkraft wird in diesem Ausführungsbeispiel allein über die Länge der Stege abgebildet. Während die ersten Stege 4 eine Länge L1 haben, haben die zweiten Stege 4a eine demgegenüber vergrößerte Länge L2. Aufgrund des so verkürzen auf die Bögen 3, 3a wirkenden Hebel ist die Steifigkeit der zweiten Zirkumferenzsegmente 22 herabgesetzt. Ein Verhältnis L2 / L1 beträgt hier etwa 3/2, kann aber auch größer oder kleiner gewählt werden, je nach Anwendungsfall. Hierdurch stellen sich auch wiederum verschiedene Zellengrößen der ersten und zweiten Zellen 40, 42 ein. Insofern kann dieses Ausführungsbeispiel auch so beschrieben werden, dass die zweiten Zirkumferenzsegmente 22 größere Zellen bilden, als die ersten Zirkumferenzsegmente 20.

Ein weiterer Unterschied zu den Ausführungsbeispielen der Figuren 6a bis 6c und 7a bis 7c liegt darin, dass die ersten Zirkumferenzsegmente 20 jeweils vier Paare an ersten Stegen 4 und so auch vier erste Zellen 40 aufweisen, während in den Ausführungsbeispielen der Figuren 6a bis 6c und 7a bis 7c jeweils nur drei vorgesehen waren.

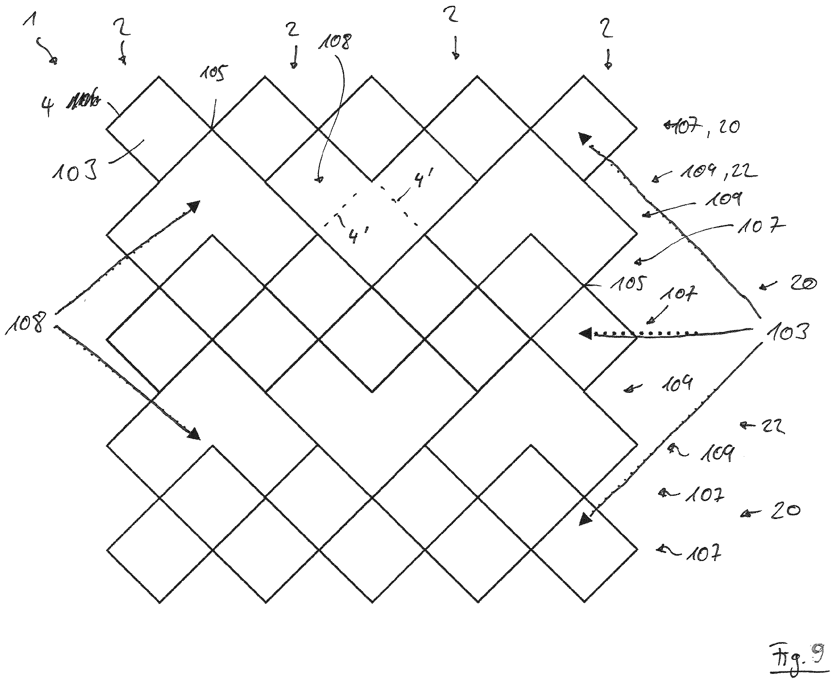

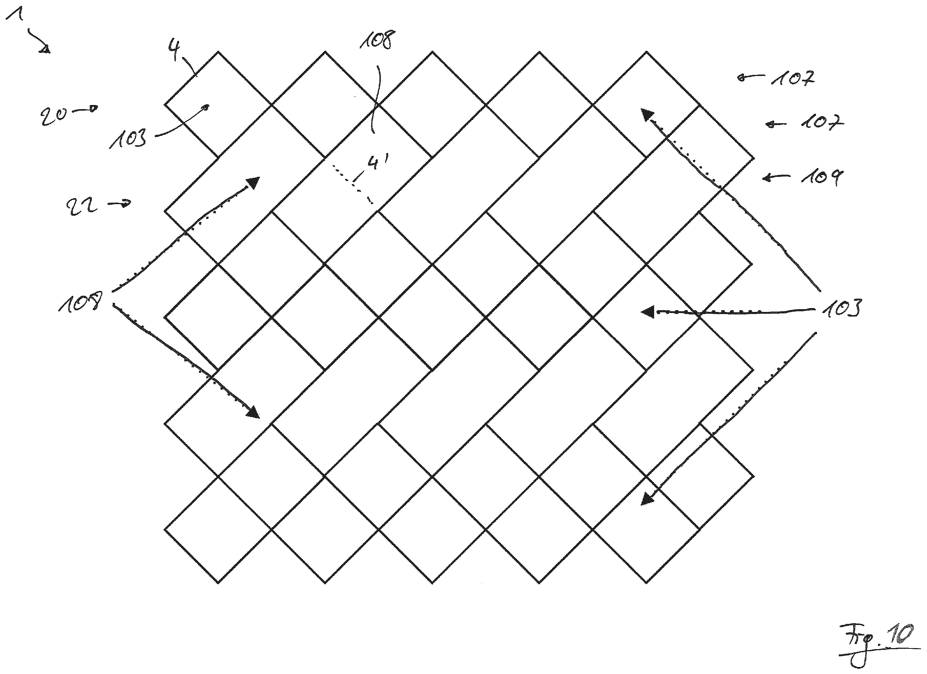

Die Figuren 9 und 10 zeigen nun alternative Ausführungsbeispiele des Stents 1 mit gestuftem Radialkraftprofil. Im Unterschied zu den vorhergehenden Ausführungsbeispielen sind allerdings alle Stege gleich breit und mit gleichem Querschnitt ausgebildet.

Figur 9 zeigt einen erfindungsgemäßen Stent 1 als Prinzipskizze in ausgebreiteten Zustand, d.h. wieder nur die Umfangsoberfläche. Der Stent 1 ist ringförmig und die Zentralachse verläuft in Figur 9 horizontal. Die Zirkumferenz wird von mäandrierenden Ringsegmenten 2 gebildet, die in axialer Richtung über mit Eckpunkten 105 verbundenen Stegen 4 eine Abfolge von kleinen Zellen 103 ausbilden. In axialer Richtung wird der Stent 1 im dargestellten Fall durch in drei Reihen 107 angeordneten kleinen Zellen 103 aus jeweils vier Stegen 4 definiert.

Im Unterschied zu den vorherigen Ausführungsbeispielen werden also Ringsegmente 2 nicht von einer durchgehenden Zickzack-Form an Stäben 4 gebildet, vielmehr sind aus dem vollständen Netz aus Stäben 4 mit Zacken 24, 26 selektiv Stäbe 4 entnommen. Bei einer regulären Zickzack-Form werden Zellen aus je vier Stäben 4 gebildet, wie dies bei der Zelle 103 in der linken oberen Ecke der Figur 9 der Fall ist. Derartige Zellen sind auch bei den vorherigen Ausführungsbeispielen vorgesehen.

Zwischen den Ringsegmenten 2 und den Reihen 107 sind jeweils große Zellen 108 angeordnet, die im dargestellten Fall von jeweils acht Stegen 4 gebildet werden. Die großen Zellen 108 sind winkelig bzw. L-förmig ausgebildet und entsprechen in ihrer Größe drei im Winkel angeordneten kleinen Zellen 103. In einer anderen Ausführungsvariante ist es durchaus möglich, die großen Zellen 108 auch rechteckig, sechseckig oder quadratisch auszubilden und dadurch die Radialkraft zu variieren. In jedem Fall können zwischen den Reihen 107 aus kleinen Zellen 103 weitere kleine Zellen 103 angeordnet sein, die Einfluss auf die Radialkraft des Stents 1 haben.

Die großen Zellen 108 sind dadurch gebildet, dass einzelne Stege 4 aus dem regelmäßigen im allgemeinen rautenförmigen Grundmuster weggelassen sind. Zwei weggelassene Stege 4' sind bei einer großen Zelle 108 in der Mitte mit gestrichelten Linien angezeigt.

Das gestufte Radialkraftprofil kommt bei dem hier gezeigten Ausführungsbeispiel wie folgt zustande: Die individuellen Stege 4, die in axialer Richtung benachbart sind, wirken als parallel-geschaltete Federelemente in Umfangsrichtung und damit in Richtung der Radialkraft. In vollständigen Reihen 107 sind in dem hier gezeigten Ausführungsbeispiel axial benachbart, also parallel-geschaltet, je zehn Stege 4 vorgesehen. In unvollständigen Reihen 109 sind in axialer Richtung benachbart lediglich vier Stege 4 vorgesehen. Hierdurch ist die durch die Stege 4 bereitgestellte Federkraft um 60% reduziert, sodass auf diese Weise verschiedene dauerhafte Auswärtskräfte COF erzeugt werden können.

In dem Ausführungsbeispiel der Figur 9 bilden in Umfangsrichtung zwei benachbarte vollständige Reihen 107 ein erstes Zirkumferenzsegment 20, und zwei unvollständige Reihen 109 ein zweites Zirkumferenzsegment 22.Figur 10 zeigt eine Variante des Stents 1 von Figur 9 mit gegenüber Figur 9 verkleinerten großen Zellen 108. Die großen Zellen 108 sind von jeweils vier gleich langen Stegen 4 begrenzt. Jedes Ringsegment 2 weist die gleiche Anzahl von großen Zellen 108 auf, im dargestellten Fall zwei.

In einer vollständigen Reihe 107 sind hier wiederum zehn Stege 4 vorgesehen, während in unvollständigen Reihen 109 insgesamt sechs Stege 4 vorgesehen sind. D.h. die Federkraft der parallel-geschalteten Stege 4 ist hier nur um 40% reduziert, wodurch eine im Vergleich zur Figur 9 höhere zweite dauerhafte Auswärtskraft COF2 erreicht wird.

Ein erfindungsgemäßer Stent 1 wird vorzugsweise aus einem Rohrstück herausgeschnitten. Das Rohrstück ist vorzugsweise aus einem Nitinol-Werkstoff und besitzt Formgedächtniseigenschaften. Die Figuren 1, 6a, 7a, 8a zeigen nicht nur den gecrimpten Zustand, sondern auch den Ausgangszustand nach dem Schneiden, vorzugsweise Laserschneiden. Das heißt, beim Herstellen des Stents 1 wird dieser in dem in den Figuren 1, 6a, 7a und 8a gezeigten Ausgangszustand aus dem Rohrstück geschnitten. Anschließend werden mehrere Schritte der Formgebung durchgeführt. Hierzu sind die zweiten Zirkumferenzsegmente zunächst mechanisch verschlossen, die ersten Zirkumferenzsegmente sind mechanisch freigegeben. Mittels eines Werkzeugs wird dann der Stent 1 aufgedehnt. Da die zweiten Zirkumferenzsegmente mechanisch verschlossen sind, werden lediglich die ersten Zirkumferenzsegmente aufgedehnt und in Form gebracht. Nach diesem ersten Schritt der Formgebung hat der Stent 1 die in den Figuren 6b, 7b und 8b gezeigte Konfiguration. Anschließend wird der mechanische Verschluss der zweiten Zirkumferenzsegmente aufgehoben. Mit demselben Werkzeug oder einem weiteren Werkzeug wird der Stent 1 dann weiter aufgedehnt. Hierbei werden dann im Wesentlichen die zweiten Zirkumferenzsegmente in Form gebracht. Die hierzu notwendige Kraft ist deutlich geringer als die zum Aufdehnen der ersten Zirkumferenzsegmente notwendige Kraft.

Der mechanische Verschluss kann klemmend, also reibschlüssig, formschlüssig oder/oder Stoffschlüssig wirken. In Figur 1 sind mit 8 Klammern bezeichnet, die einen mechanischen Verschluss für die zweiten Zirkumferenzsegmente bilden. Alternativ oder zusätzlich können auch Materialbrücken zwischen den Stegen der zweiten Zirkumferenzsegmente 22 vorgsehen sein, die beim ersten Schritt der Formgebung ein Aufdehnen der zweiten Zirkumferenzsegmente entgegenwirken. Diese Materialbrücken sind dann vor dem Durchführen des zweiten Formgebungsschritts zu entfernen.

Die Anmeldung beinhaltet zusätzlich folgende Ausführungsbeispiele:
1. Selbstexpandierender flexibler intravaskulärer Stent (1) zum Einsatz in ein Gefäß, mit
   zwei oder mehr axial miteinander verbundenen Ringsegmenten (2, 2a), die jeweils aus einer Mehrzahl an mäanderförmig über Bögen (7) miteinander verbundener Stegen (5, 5a) gebildet sind,
   wobei der Stent (1) einen gecrimpten Zustand mit reduziertem Durchmesser und einen expandierten Zustand mit einem Nominaldurchmesser (DN) hat, und
   die Ringsegmente (2, 2a) in einem ersten Durchmesserbereich (D1) kleiner als der Nominaldurchmesser (DN) eine erste Expansionscharakteristik aufweisen, und
   in einem zweiten Durchmesserbereich (D2) größer als der Nominaldurchmesser (DN) eine zweite Expansionscharakteristik aufweisen, die verschieden ist von der ersten Expansionscharakteristik.
2. Flexibler intravaskulärer Stent nach Ausführungsbeispiel 1, wobei der zweite Durchmesserbereich wenigstens 10% eines Maximaldurchmessers beträgt.
3. Flexibler intravaskulärer Stent nach Ausführungsbeispiel 1 oder 2, wobei
   die Ringsegmente (2, 2a) in einem ersten Durchmesserbereich (D1) kleiner als der Nominaldurchmesser (DN) eine erste Radial-Steifigkeit aufweisen, und
   in einem zweiten Durchmesserbereich (D2) größer als der Nominaldurchmesser (DN) eine zweite Radial-Steifigkeit aufweisen,
   wobei die zweite Radial-Steifigkeit vorzugsweise geringer ist als die erste Radial-Steifigkeit.
4. Flexibler intravaskulärer Stent nach einem der vorstehenden Ausführungsbeispiele, aufweisend einen ersten Radialkraft-Abfall in dem ersten Durchmessebereich (D1) bis zum Nominaldurchmesser (DN) und einen zweiten Radialkraftabfall in dem zweiten Durchmessebereich (D2).
5. Flexibler intravaskulärer Stent nach Ausführungsbeispiel 4, wobei die Radialkraft (F1, F2) in dem ersten Durchmessebereich (D1) beim Expandieren auf ein erstes Radialkraftniveau (FN1) abfällt und im zweiten Durchmesserbereich (D2) auf ein zweites Radialkraftniveau (FN2) abfällt, das geringer ist als das erste Radialkraftniveau (FN1).
6. Flexibler intravaskulärer Stent nach einem der vorstehenden Ausführungsbeispiele, wobei ein Radialkraft-Diameter-Profil des Stents (1) einen Knick (K1) oder Sprung aufweist.
7. Flexibler intravaskulärer Stent nach einem der vorstehenden Ausführungsbeispiele, wobei ein Radialkraft-Diameter-Profil des Stents (1) im ersten Durchmesserbereich (D1) ausgehend vom gecrimpten Zustand beim Expandieren zunächst einen Abschnitt mit einer ersten Steigung (S1), dann einen Abschnitt mit einer zweiten Steigung (S2), und im zweiten Durchmesserbereich (D2) ausgehend vom Nominaldurchmesser (DN) einen dritten Abschnitt mit einer dritten Steigung (S3) und einen vierten Abschnitt mit einer vierten Steigung (S4) aufweist, wobei die erste Steigung (S1) größer als die zweite Steigung (S2) und die vierte Steigung (S4) ist, und die dritte Steigung (S3) größer als die zweite Steigung (S2) und die vierte Steigung (S4) ist.
8. Flexibler intravaskulärer Stent nach einem der vorstehenden Ausführungsbeispiele, wobei ein Radialkraft-Diameter-Profil des Stents (1) ausgehend vom gecrimpten Zustand bis zum expandierten Zustand folgenden Verlauf nimmt: im ersten Durchmesserbereich (D1) zunächst eine Steigungsabnahme gefolgt von einer Steigungszunahme, dann beim Übertritt in den zweiten Durchmesserbereich (D2) eine erneute Steigungsabnahme und vorzugsweise abschließend erneute Steigungszuname.
9. Flexibler intravaskulärer Stent nach einem der vorstehenden Ausführungsbeispiele, wobei ein Radialkraft-Diameter-Profil des Stents (1) wenigstens einen, vorzugsweise zwei Wendepunkte aufweist.
10. Flexibler intravaskulärer Stent nach einem der vorstehenden Ausführungsbeispiele, wobei der erste Durchmesserbereich (D1) einen ersten Radial-Steifigkeitsprofilabschnitt definiert und der erste Radial-Steifigkeitsprofilabschnitt degressiv oder linear ist.
11. Flexibler intravaskulärer Stent nach einem der vorstehenden Ausführungsbeispiele, wobei der zweite Durchmesserbereich (D2) einen zweiten Radial-Steifigkeitsprofilabschnitt definiert und der zweite Radial-Steifigkeitsprofilabschnitt degressiv oder linear ist.
12. Flexibler intravaskulärer Stent nach Ausführungsbeispiel 10 und 11, wobei der zweite Radial-Steifigkeitsprofilabschnitt stärker degressiv ist oder eine größere Steigung hat als der erste Radial-Steifigkeitsprofilabschnitt.
13. Flexibler intravaskulärer Stent nach einem der vorstehenden Ausführungsbeispiele, wobei der Stent (1) in dem ersten Durchmesserbereich (D1) eine erste dauerhafte Auswärtskraft (chronic outward force, COF1) in einem ersten Radialkraftbereich bereitstellt und in dem zweiten Durchmesserbereich (D2) eine zweite dauerhafte Auswärtskraft (chronic outward force, COF2) in einem zweiten Radialkraftbereich bereitstellt, wobei die erste dauerhafte Auswärtskraft COF1 um einen Faktor von wenigstens 2, vorzugsweise 4, größer ist als die zweite dauerhafte Radialkraft COF2 oder die zweite dauerhafte Radialkraft (chronic outward force, cof) COF2 nahezu 0 beträgt.
14. Flexibler intravaskulärer Stent nach einem der vorstehenden Ausführungsbeispiele, wobei jedes Ringsegment (2, 2a) über die Ringzirkumferenz verteilt eine Mehrzahl an ersten Zirkumferenzsegmenten (20) und eine Mehrzahl an zweiten Zirkumferenzsegmenten (22) aufweist, die sich strukturell unterscheiden.
15. Flexibler intravaskulärer Stent nach Ausführungsbeispiel 14, wobei die ersten und zweiten Zirkumferenzsegmente (20, 22) gemeinsam eine erste dauerhafte Auswärtskraft (COF1) in dem ersten Durchmesserbereich (D1) definieren und die zweiten Zirkumferenzsegmente (22) eine zweite dauerhafte Auswärtskraft (COF2) in dem zweiten Durchmesserbereich (D2) definieren.
16. Flexibler intravaskulärer Stent nach Ausführungsbeispiel 14 oder 15, wobei die ersten Zirkumferenzsegmente (20) und zweiten Zirkumferenzsegmente (22) benachbarter Ringsegmente (2, 2a) jeweils axial zueinander ausgerichtet sind.
17. Flexibler intravaskulärer Stent nach einem der vorstehenden Ausführungsbeispiele 14 bis 16, wobei die Stege (4) der ersten Zirkumferenzsegmente (20, 5) eine größere Stärke aufweisen, als die Stege (4a) der zweiten Zirkumferenzsegmente (22, 5a).
18. Flexibler intravaskulärer Stent nach Ausführungsbeispiel 17, dadurch gekennzeichnet, dass die Steifigkeit der Stege (4, 4a) durch die Stegbreite definiert ist., derart, dass erste Stege (4) der ersten Zirkumferenzsegmente (20) eine erste Stegbreite (b1) und zweite Stege (4a) der zweiten Zirkumferenzsegmente (22) eine zweite Stegbreite (b2) aufweisen, die geringer ist als die erste Stegbreite (b1).
19. Flexibler intravaskulärer Stent nach einem der Ausführungsbeispiele 14 bis 18, gekennzeichnet durch wenigstens zwei zweite Zirkumferenzsegmente pro Ringsegment.
20. Flexibler intravaskulärer Stent nach einem der vorstehenden Ausführungsbeispiele 14 bis 19, dadurch gekennzeichnet, dass die zweiten Zirkumferenzsegmente (5a) spiralförmig versetzt über die Länge des Stents (1) angeordnet sind.
21. Flexibler intravaskulärer Stent nach einem der Ausführungsbeispiele 14 bis 19, dadurch gekennzeichnet, dass die zweiten Zirkumferenzsegmente (5a) linear in axialer Richtung des Stents (1) angeordnet sind.
22. Flexibler intravaskulärer Stent nach einem der vorstehenden Ausführungsbeispiele, dadurch gekennzeichnet, dass er aus einer Formgedächtnislegierung besteht.
23. Flexibler intravaskulärer Stent nach Ausführungsbeispiel 22, dadurch gekennzeichnet, dass er aus einer Nickel-Titan-Legierung besteht, vorzugsweise aus Nitinol.
24. Flexibler intravaskulärer Stent nach einem der vorstehenden Ausführungsbeispiele, dadurch gekennzeichnet, dass Ringsegmente (2, 2a) über Verbindungsstege mit benachbarten Ringsegmenten (2, 2a) verbunden sind.
25. Flexibler intravaskulärer Stent nach Ausführungsbeispiel 24, dadurch gekennzeichnet, dass die Verbindungsstege die Bögen benachbarter Ringsegmente (2, 2a) miteinander verbinden.
26. Flexibler intravaskulärer Stent nach einem der vorstehenden Ausführungsbeispiele, dadurch gekennzeichnet, dass wenigstens eines der randständigen Ringsegmente (2, 2a) mit dem benachbarten Ringsegment (2) unmittelbar verbunden ist.
27. Flexibler intravaskulärer Stent nach einem der Ausführungsbeispiele 24 oder 25, dadurch gekennzeichnet, dass die Verbindungsstege (3) eine einheitliche Stegbreite aufweisen.
28. Flexibler intravaskulärer Stent nach Ausführungsbeispiel 9, wobei die Ringsegmente (2) eine Vielzahl von Zellen (103, 108) ausbilden, wobei der Stent (1) große Zellen (108) und kleine Zellen (103) aufweist, wobei die großen Zellen (108) zweite Zirkumferenzsegmente (22) und die kleinen Zellen (103) erste Zirkumferenzsegmente (20) ausbilden.
29. Flexibler intravaskulärer Stent nach Ausführungsbeispiel 28, wobei kleine Zellen (103) in axialer Richtung des Stents (1) aneinander grenzend aufgereiht sind, aus jeweils vier Stegen (4) gebildet sind und über ihre Eckpunkte (5) miteinander verbunden sind, große Zellen (108) zwischen den Reihen (107) aus kleinen Zellen (103) angeordnet sind, wobei die großen Zellen (108) von jeweils wenigstens sechs Stegen (4) gebildet werden, und die kleinen Zellen (103) bei der Expansion des Stents (1) den Nominaldurchmesser (DN) definieren und die großen Zellen (108) eine Expansionsreserve (E) bilden.
30. Flexibler intravaskulärer Stent nach Ausführungsbeispiel 28 oder 29, aufweisend wenigstens drei Reihen in axialer Richtung angeordnete kleine Zellen (103).
31. Flexibler intravaskulärer Stent nach einem der Ausführungsbeispiele 28 bis 30, dadurch gekennzeichnet, dass zwischen den Ringsegmenten (2, 2a) jeweils wenigstens zwei große Zellen (108) angeordnet sind.
32. Flexibler intravaskulärer Stent nach einem der vorstehenden Ausführungsbeispiele 28 bis 31, dadurch gekennzeichnet, dass die großen Zellen (108) spiralförmig versetzt über die Länge des Stents (1) angeordnet sind.
33. Flexibler intravaskulärer Stent nach einem der Ausführungsbeispiele 28 bis 31, dadurch gekennzeichnet, dass die großen Zellen (108) linear in axialer Richtung des Stents (1) angeordnet sind.
34. Flexibler intravaskulärer Stent nach einem der Ausführungsbeispiele 28 bis 33, dadurch gekennzeichnet, dass die Stege (4) des Stents (1) eine einheitliche Länge (L) aufweisen.
35. Verwendung eines flexiblen intravaskulären Stents nach einem der vorstehenden Ausführungsbeispiele zur Variation der Radialkraft zur Erzeugung eines gestuften Radialkraftprofils.
36. Herstellungsverfahren für einen Stent (1), vorzugsweise nach einem der Ausführungsbeispiele 1 bis 34, mit den Schritten:
   - Bereitstellen eines Rohrstücks aus einem Formgedächtnismaterial;
   - Schneiden des Stents (1) aus dem Rohrstück, wobei der Stent (1) erste und zweite Zirkumferenzsegmente (20, 22) aufweist;
   - mechanisches Verschließen der zweiten Zirkumferenzsegmente;
   - Durchführen eines ersten Formgebungsschritts durch mechanisches Aufdehnen der ersten Zirkumferenzsegmente;
   - im Anschluss hieran: Lösen des mechanischen Verschlusses der zweiten Zirkumferenzsegmente und Durchführen eines zweiten Formgebungsschritts durch mechanisches Aufdehnen der zweiten Zirkumferenzsegmente.
37. Herstellungsverfahren nach Ausführungsbeispiel 36, wobei das mechanisches Verschließen der zweiten Zirkumferenzsegmente umfasst: Einsetzen eines mechanischen Haltemittels, insbesondere einer Klammer, zum mechanischen Verschließen der zweiten Zirkumferenzsegmente.
38. Herstellungsverfahren nach Ausführungsbeispiel 36, wobei das mechanisches Verschließen der zweiten Zirkumferenzsegmente umfasst: Einbringen von Materialbrücken zwischen Stege der zweiten Zirkumferenzsegmente, vorzugsweise während des Schritts des Schneidens.

## Patentansprüche

1. Selbstexpandierender flexibler intravaskulärer Stent (1) zum Einsatz in ein Gefäß, mit
zwei oder mehr axial miteinander verbundenen Ringsegmenten (2, 2a), die jeweils aus einer Mehrzahl an mäanderförmig über Bögen (7) miteinander verbundener Stegen (5, 5a) gebildet sind,
wobei der Stent (1) einen gecrimpten Zustand mit reduziertem Durchmesser und einen expandierten Zustand mit einem Nominaldurchmesser (DN) hat, und
die Ringsegmente (2, 2a) in einem ersten Durchmesserbereich (D1) kleiner als der Nominaldurchmesser (DN) eine erste Expansionscharakteristik aufweisen, und
in einem zweiten Durchmesserbereich (D2) größer als der Nominaldurchmesser (DN) eine zweite Expansionscharakteristik aufweisen, die verschieden ist von der ersten Expansionscharakteristik,
wobei der Stent einstückig und aus einem Rohr hergestellt ist.

2. Flexibler intravaskulärer Stent nach Anspruch 1, wobei der zweite Durchmesserbereich wenigstens 10% eines Maximaldurchmessers beträgt.

3. Flexibler intravaskulärer Stent nach einem der vorstehenden Ansprüche, wobei ein Radialkraft-Diameter-Profil des Stents (1) einen Knick (K1) oder Sprung aufweist.

4. Flexibler intravaskulärer Stent nach einem der vorstehenden Ansprüche, wobei ein Radialkraft-Diameter-Profil des Stents (1) wenigstens einen, vorzugsweise zwei Wendepunkte aufweist.

5. Flexibler intravaskulärer Stent nach einem der vorstehenden Ansprüche, wobei der Stent (1) in dem ersten Durchmesserbereich (D1) eine erste dauerhafte Auswärtskraft (chronic outward force, COF1) in einem ersten Radialkraftbereich bereitstellt und in dem zweiten Durchmesserbereich (D2) eine zweite dauerhafte Auswärtskraft (chronic outward force, COF2) in einem zweiten Radialkraftbereich bereitstellt, wobei die erste dauerhafte Auswärtskraft COF1 um einen Faktor von wenigstens 2, vorzugsweise 4, größer ist als die zweite dauerhafte Radialkraft COF2 oder die zweite dauerhafte Radialkraft (chronic outward force, cof) COF2 nahezu 0 beträgt.

6. Flexibler intravaskulärer Stent nach einem der vorstehenden Ansprüche, wobei jedes Ringsegment (2, 2a) über die Ringzirkumferenz verteilt eine Mehrzahl an ersten Zirkumferenzsegmenten (20) und eine Mehrzahl an zweiten Zirkumferenzsegmenten (22) aufweist, die sich strukturell unterscheiden.

7. Flexibler intravaskulärer Stent nach Anspruch 6, wobei die Stege (4) der ersten Zirkumferenzsegmente (20, 5) eine größere Stärke aufweisen, als die Stege (4a) der zweiten Zirkumferenzsegmente (22, 5a).

8. Flexibler intravaskulärer Stent nach Anspruch 7, **dadurch gekennzeichnet, dass** die Steifigkeit der Stege (4, 4a) durch die Stegbreite definiert ist., derart, dass erste Stege (4) der ersten Zirkumferenzsegmente (20) eine erste Stegbreite (b1) und zweite Stege (4a) der zweiten Zirkumferenzsegmente (22) eine zweite Stegbreite (b2) aufweisen, die geringer ist als die erste Stegbreite (b1).

9. Flexibler intravaskulärer Stent nach einem der vorstehenden Ansprüche, **dadurch gekennzeichnet, dass** er aus einer Formgedächtnislegierung besteht.

10. Flexibler intravaskulärer Stent nach einem der vorstehenden Ansprüche, **dadurch gekennzeichnet, dass** Ringsegmente (2, 2a) über Verbindungsstege mit benachbarten Ringsegmenten (2, 2a) verbunden sind.

11. Flexibler intravaskulärer Stent nach einem der vorstehenden Ansprüche, **dadurch gekennzeichnet, dass** wenigstens eines der randständigen Ringsegmente (2, 2a) mit dem benachbarten Ringsegment (2) unmittelbar verbunden ist.

12. Flexibler intravaskulärer Stent nach Anspruch 4, wobei die Ringsegmente (2) eine Vielzahl von Zellen (103, 108) ausbilden, wobei der Stent (1) große Zellen (108) und kleine Zellen (103) aufweist, wobei die großen Zellen (108) zweite Zirkumferenzsegmente (22) und die kleinen Zellen (103) erste Zirkumferenzsegmente (20) ausbilden.

13. Flexibler intravaskulärer Stent nach Anspruch 12, wobei kleine Zellen (103) in axialer Richtung des Stents (1) aneinander grenzend aufgereiht sind, aus jeweils vier Stegen (4) gebildet sind und über ihre Eckpunkte (5) miteinander verbunden sind, große Zellen (108) zwischen den Reihen (107) aus kleinen Zellen (103) angeordnet sind, wobei die großen Zellen (108) von jeweils wenigstens sechs Stegen (4) gebildet werden, und die kleinen Zellen (103) bei der Expansion des Stents (1) den Nominaldurchmesser (DN) definieren und die großen Zellen (108) eine Expansionsreserve (E) bilden.

14. Verwendung eines flexiblen intravaskulären Stents nach einem der vorstehenden Ansprüche zur Variation der Radialkraft zur Erzeugung eines gestuften Radialkraftprofils.

15. Herstellungsverfahren für einen Stent (1) nach einem der Ansprüche 1 bis 13, mit den Schritten:
- Bereitstellen eines Rohrstücks aus einem Formgedächtnismaterial;
- Schneiden des Stents (1) aus dem Rohrstück, wobei der Stent (1) erste und zweite Zirkumferenzsegmente (20, 22) aufweist;
- mechanisches Verschließen der zweiten Zirkumferenzsegmente;
- Durchführen eines ersten Formgebungsschritts durch mechanisches Aufdehnen der ersten Zirkumferenzsegmente;
- im Anschluss hieran: Lösen des mechanischen Verschlusses der zweiten Zirkumferenzsegmente und Durchführen eines zweiten Formgebungsschritts durch mechanisches Aufdehnen der zweiten Zirkumferenzsegmente.
